# EUROPEAN PATENT APPLICATION

(11) **EP 4 606 320 A1**
(43) Date of publication of application: **27.08.2025**
(21) Application number: 25159077.4
(22) Date of filing: 20.02.2025
(51) Int. Cl.: A61B 17/02, A61B 17/00

(54) **NEUROMONITORING DILATORS, PROBES, AND CANNULATED BLADES FOR MODULAR TISSUE RETRACTOR**

(30) Priority: 23.02.2024 US 202463557124 P; 11.02.2025 US 202519050710
(71) Applicant: Warsaw Orthopedic, Inc., Warsaw, IN 46581 (US)
(72) Inventor: JOSSE, Loic, Palm Beach Garden (US); BALLARD, Rodney, Lakeland, 38002 (US)
(74) Representative: Viering, Jentschura & Partner mbB Patent- und Rechtsanwälte

(57) **Abstract**

A tissue retraction and neuro monitoring system includes one or more dilators each having a conductive element, wherein the conductive element is capable of neuro monitoring. At least one of the dilators includes a tip configured to penetrate patient anatomy and a surface configured to promote penetration of the one or more dilators into muscle tissue while preventing penetration of the one or more dilators into spinal disc tissue. The system also includes a first blade and a second blade, each having a respective blade shaft. The system also includes a third blade having a posterior blade shaft with a slot on a lateral side thereof, the posterior blade shaft being configured to house a discal shim in a cavity thereof, wherein the discal shim is pre-loaded within the cavity of the third blade.

## Description

### BACKGROUND

Spinal disorders such as degenerative disc disease, disc herniation, osteoporosis, spondylolisthesis, stenosis, scoliosis and other curvature abnormalities, kyphosis, tumor, and fracture may result from factors including trauma, disease and degenerative conditions caused by injury and aging. Spinal disorders typically result in symptoms including pain, nerve damage, and partial or complete loss of mobility.

Non-surgical treatments, such as medication, rehabilitation and exercise can be effective, how-ever, may fail to relieve the symptoms associated with these disorders. Surgical treatment of these spinal disorders includes fusion, fixation, discectomy, laminectomy and implantable prosthetics. Surgical retractors may be employed during a surgical treatment to provide access and visualization of a surgical site. Such retractors space apart and support tissue and/or other anatomical structures to expose anatomical structures at the surgical site and/or provide a surgical pathway for the surgeon to the surgical site.

Neuromonitoring and positional monitoring of surgical tools during surgical procedures are helpful to prevent damage to the nervous system and to ensure proper placement of the surgical tools. Therefore, there is a need for a tissue retraction system having neuromonitoring and positional monitoring capability.

### SUMMARY

This disclosure describes a tissue retraction and neuro monitoring system. The system may use any of the variously disclosed blades, extendable blades, probes and dilators. Additionally this disclosure describes a method of using a tissue retraction and neuro monitoring system.
In an aspect, the disclosure describes a tissue retraction and neuro monitoring system. The system includes one or more dilators each having a conductive element, wherein the conductive element is capable of neuro monitoring. At least one of the dilators includes a tip configured to penetrate patient anatomy. A distal end of each of the one or more dilators includes a surface configured to promote penetration of the one or more dilators into muscle tissue while preventing penetration of the one or more dilators into spinal disc tissue. The system also includes a first blade and a second blade, each having a respective blade shaft and a third blade having a posterior blade shaft with a slot on a lateral side thereof, the posterior blade shaft being configured to house a discal shim in a cavity thereof, wherein the discal shim is pre-loaded within the cavity of the third blade and configured to allow a user to impact the discal shim into patient disc space while the one or more dilators are positioned in nearby patient anatomy.

In another aspect the disclosure describes a tissue retraction and neuro monitoring system. The system includes one or more dilators each having a conductive element, wherein the conductive element is capable of neuro monitoring, wherein at least one of the dilators includes a tip configured to penetrate patient anatomy and, wherein a distal end of each of the one or more dilators includes a surface configured to promote penetration of the one or more dilators into muscle tissue while preventing penetration of the one or more dilators into spinal disc tissue. The system also includes a first blade and a second blade, each having a respective blade shaft and a third blade having a posterior blade shaft with a slot on a lateral side thereof, the posterior blade shaft being configured to house a discal shim in a cavity thereof, wherein the discal shim is pre-loaded to allow a user to impact the discal shim into patient disc space while the one or more dilators are positioned in nearby patient anatomy. The slot of the third blade is configured to receive an insulated probe having a conductive tip, so that the conductive tip is exposed to patient anatomy and is configured to send information to an external device during a surgical procedure.

In still another aspect, a method of using a tissue retraction and neuro monitoring system. The methods includes inserting an initial dilator capable and one or more probes of neuro monitoring into patient disc space, inserting at least one additional dilator capable of neuro monitoring sequentially into patient disc space, inserting one or more tissue retractors while the initial dilator and at least one additional dilator are in place in the patient disc space, impacting an extendable blade of a discal shim with a discal shim pusher, securing the one or more tissue retractors to a surgical table, removing the initial dilator and the at least one additional dilators and retracting patient tissue with the one or more tissue retractors while the one or more probes perform neuro monitoring.

### BRIEF DESCRIPTION OF THE DRAWINGS

FIG. 1 is a perspective view of a blade adjustment and positioning tool.
FIG. 2 is an exploded parts view of a blade adjustment and positioning tool.
FIG. 3 is a perspective view of the blade adjustment and positioning tool engaged with a modular blade and an extendable blade.
FIG. 4 is a perspective view of the blade adjustment and positioning tool engaged with a modular blade and an extendable blade.
FIG. 5 is a perspective view of the inside surfaces of a modular blade and an extendable blade having a footed tip at the distal end thereof.
FIG. 6 is a perspective view of the modular blade and extendable blade of FIG. 5.
FIG. 7 is a perspective view of a quick connect handle.
FIG. 8 is an exploded parts view of a quick connect handle.
FIG. 9 is a perspective view of a retractor mount coupler.
FIG. 10 is a perspective view of the modular and extendable blades of FIGS. 5-6 coupled to the quick connect handle of FIGS. 7-8 and the retractor mount coupler of FIG. 9.
FIG. 11 is a perspective view of the modular and extendable blades of FIGS. 5-6 before being coupled to the quick connect handle of FIGS. 7-8.
FIG. 12 is a first perspective view of an additional embodiment of a modular retractor.
FIG. 13 is a second perspective view of the additional embodiment of the modular retractor of FIG. 10
FIG. 14 is a top down view of the embodiment of FIGS. 10-11.
FIG. 15 is a bottom perspective view of a blade coupling portion.
FIG. 16 is an enlarged view of the embodiment of FIGS. 10-12 from a top perspective with the top cover removed for ease of understanding of the internal gear system.
FIG. 17 is an enlarged view of the embodiment of FIGS. 10-12 from a bottom perspective showing various structural features of a table mount quick connect coupler.
FIG. 18 is a perspective view of various armatures of a quick connect table mount system for supporting various retractor embodiments disclosed herein.
FIG. 19 is a first perspective view of a quick connect coupler for connecting various retractor embodiments to various quick connect table mount systems disclosed herein.
FIG. 20 is a side view of the quick connect coupler of FIG. 19.
FIG. 21 is a perspective view of a modular retractor system including the quick connect couplers of FIGS. 19-20.
FIG. 22 is a top down view of the system of FIG. 21.
FIG. 23 is a perspective view of a secondary module that may be coupled and uncoupled with various primary retractor embodiments.

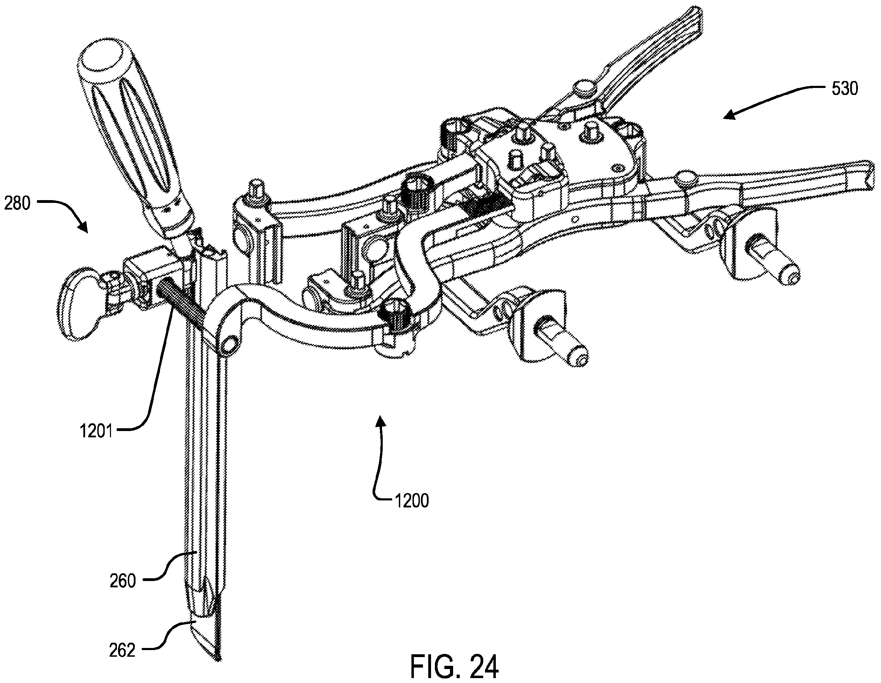

FIG. 24 is a perspective view of the secondary module of FIG. 25 coupled to a primary retractor.
FIG. 25 is a top down view of a modular retractor supporting first and second blades to be slidably coupled to an outermost dilator.
FIG. 26 is a perspective view of the three blades being slidably coupled to an outermost dilator.
FIG. 27 is a perspective view of a modular retractor system during use.
FIG. 28 is a second perspective view of a modular retractor system.
FIG. 29 is a top down view of a modular retractor system.
FIG. 30 is an enlarged top down view of a region of FIG. 129.
FIG. 31 is a top down view of a modular retractor system.
FIG. 32 is a perspective view of a pair of blades for use with various modular retractor systems disclosed herein.
FIG. 33 is a perspective view of a pin delivery cannula for use with various modular retractor systems disclosed herein.
FIG. 34 is a perspective view of a pin for use with the pin delivery cannula of FIG. 33.
FIG. 35 is a perspective view of the pin delivery cannula of FIG. 33 and the pin of FIG. 34 in an example operative configuration.
FIG. 36 is a perspective view of a support module for attaching to arm portions of various modular retractor systems disclosed herein.

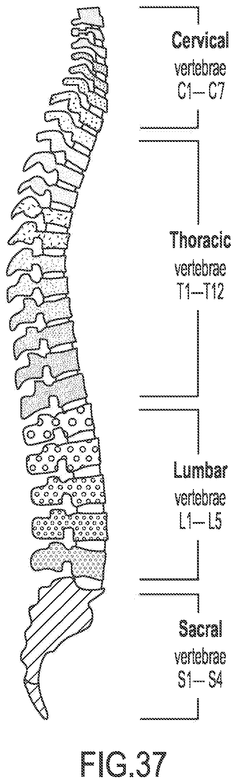

FIG. 37 is a reference drawing showing the human spine of which various disclosed implant embodiments may be installed in.

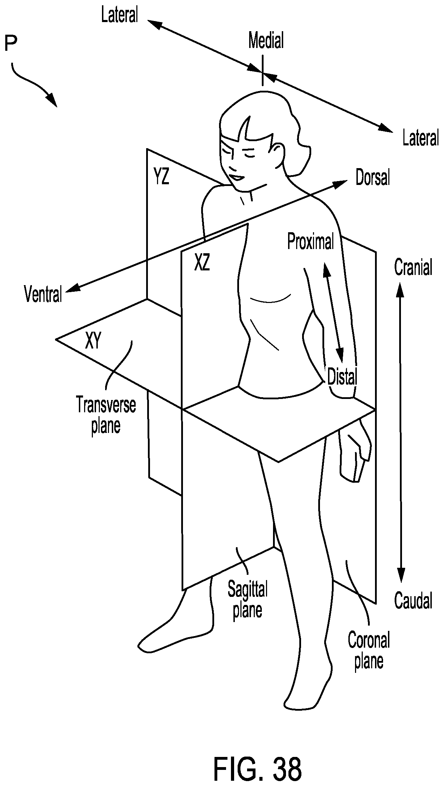

FIG. 38 is a reference drawing showing various planes and reference directions of which the various disclosed implant embodiments may move in or act in with respect to a patient.
FIG. 39 is a perspective view of a tissue retraction and neuromonitoring system.
FIG. 40 is an alternate view of a tissue retraction and neuromonitoring system.
FIG. 41A is a perspective view of a locked probe
FIG. 41B is a perspective view of a free probe
FIG. 42 is an alternate view of a tissue retraction system with a cross sectional view of a posterior blade showing a discal shim installed on the interior of the posterior blade.
FIG. 43 is a cross sectional view of a posterior blade showing a discal shim installed on the interior of the posterior blade and a discal shim pusher loaded in the discal shim.
FIG. 44 is a cross sectional view of the embodiment of FIG. 43 with a set of nested dilators loaded in the discal shim.
FIG. 45 is an alternate view of the embodiment of FIG. 44.
FIG. 46 is an example flow chart method of use of disclosed modular retractor systems.
FIG. 47 is an embodiment of a navigation guide installed on a neuro monitored dilator.

### DETAILED DESCRIPTION

U.S. Patent Application No. 16/926,123, U.S. Patent Application No., U.S. Patent Application No. 17/683,925, 17/336,860 U.S. Patent Application No. 17/710,176 are hereby incorporated by reference in their entirety.

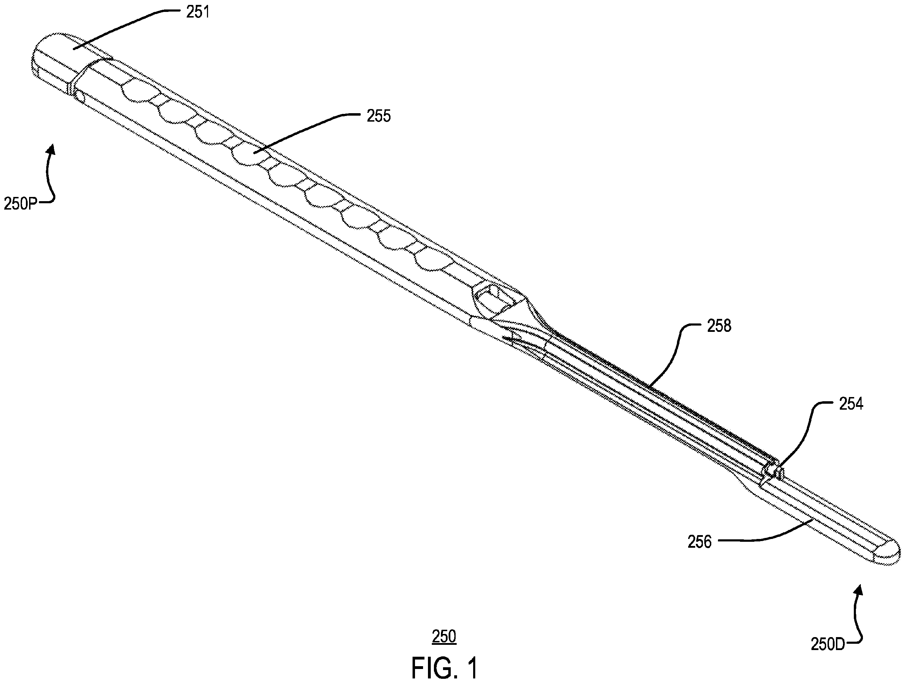

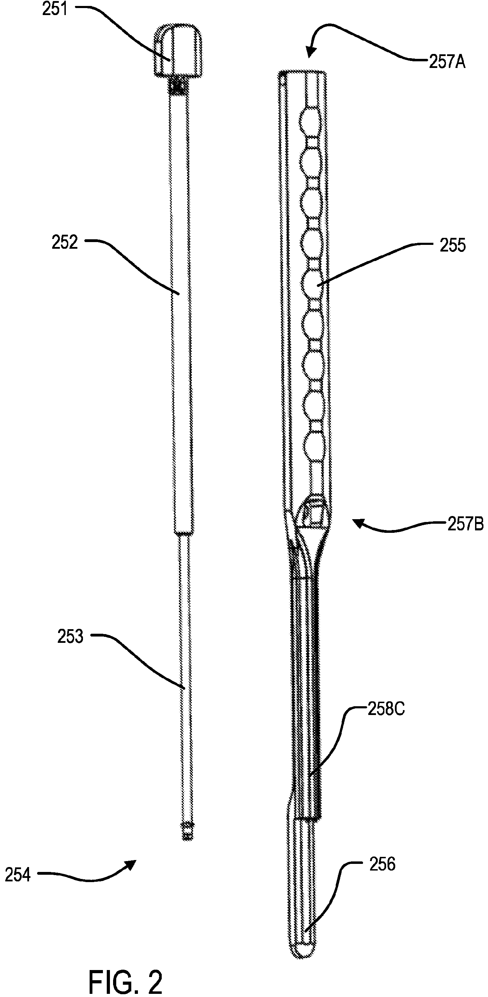

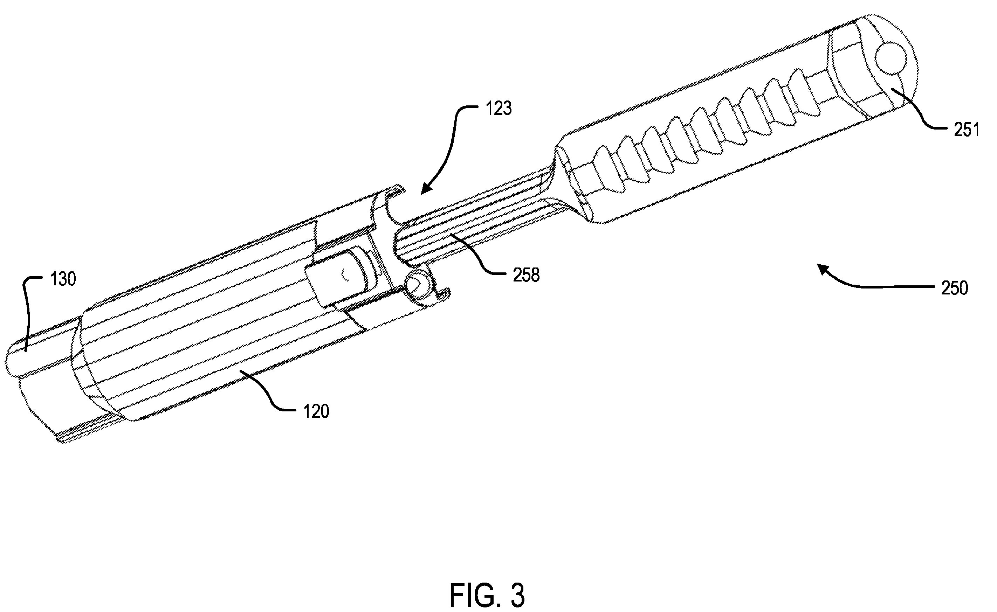

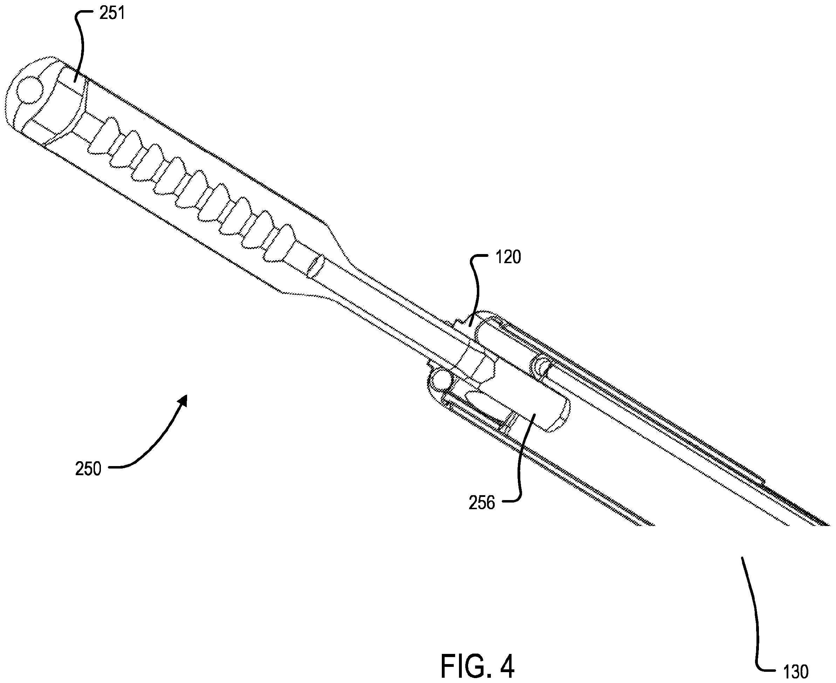

Referring generally to FIGS. 1-4 a blade adjustment and positioning tool 250 is disclosed. FIG. 1 is a perspective view of a blade adjustment and positioning tool 250 and FIG. 2 is an exploded parts view of the blade adjustment and positioning tool 250. FIG. 3 is an outside surface perspective view of the blade adjustment and positioning tool engaged with a modular blade and an extendable blade and FIG. 4 is an inside surface perspective view of the blade adjustment and positioning tool engaged with a modular blade and an extendable blade, for example. In the example embodiment, tool 250 may extend in a longitudinal direction from a proximal end 250P to a longitudinal end 250D, for example. In the example embodiment, the proximal end may be defined by a rotatable turnkey 251 and the distal end may be defined by a tab 256.

In the example embodiment, the first portion 250A may comprise a gripping portion 255 having a plurality of gripping indentations extending along a length thereof, for example. Additionally, the gripping portion may include a centrally disposed shaft extending therethrough in the longitudinal direction between proximal aperture 257A and medial aperture 257B, for example. Additionally, the second portion may include a matting rail 258 having a size and shape that corresponds to a size and shape of a channel of modular blade 120. However, it shall be understood that reference to channel 123 is by example only, and that matting rail 258 and the corresponding channel of modular blade 120 does not necessarily need indentations 125 and arcuate channel portions 123B, for example. In various embodiments, the distal end of first portion 250A may be defined by tab 256. In the example embodiment, tab 256 is offset laterally from an extension axis extending through proximal aperture 257A and medial aperture 257B, for example.

Second portion 250B may include a turnkey 251 and/or a knob at a proximal end thereof. Turnkey 251 may be coupled to or monolithically formed with primary shaft 252 and extension shaft 253. In various embodiments, extension shaft 243 may comprise a drive feature or driving head 254 at a distal end thereof. In operation, second portion 250B may be insert inside of first portion 250A by inserting the driving head 254, extension shaft 253, and primary shaft 252 through proximal aperture 257A. Due to the particular design of this embodiment, the primary shaft 252 may be rotatably disposed within the central shaft of the first portion and primary shaft 252 may have a size and shape generally corresponding to a size and shape of the central shaft of the first portion, e.g., substantially the same diameter and a length substantially the same as a distance between proximal aperture 257A and medial aperture 257B. Extension shaft 253 may be partially mated to and/or disposed within an open channel 258C of matting rail 258 such that it may freely rotate and so can driving end 254.

With reference to FIGS. 3 and 4, tool 250 may couple to a modular blade 120 by inserting matting rail 258 with channel 123. Additionally, as seen best in FIG. 4, due to the offset nature of tab 256 an inside surface of tab 256 may be directly adjacent to and/or directly contact an inside surface of extendable blade 130. In this way, tool 250 may provide a fulcrum or handle to manipulate the modular blade 120 and extendable blade 130. At least one particularly advantageous use of tool 250 may be when modular blade 120 and extendable blade 130 are coupled to a free hand module. Additionally, in some embodiments, an end user may initially mate first portion 250A to modular blade 120 and extendable blade 130, then insert second portion into first portion, and slide second portion forward in a distal direction such that it pushes extendable blade 130 forward. In some embodiments, a chiseled end of second portion, e.g., drive feature 254, may unseat a protrusion of extendable blade 130 from a corresponding indentation of modular blade 120. In this way, an end user may utilize tool 250 to extend a position of extendable blade 130 via second portion 250B and may use tool 250 as a fulcrum or handle for applying a mechanical advantage as a fulcrum to modular blade 250 and extendable blade 230, for example. Additionally, in at least some embodiments, because drive end 254 may be rotatable, it may facilitate the unseating of the protrusion of the extendable blade 130 from the corresponding indentation of modular blade 120.

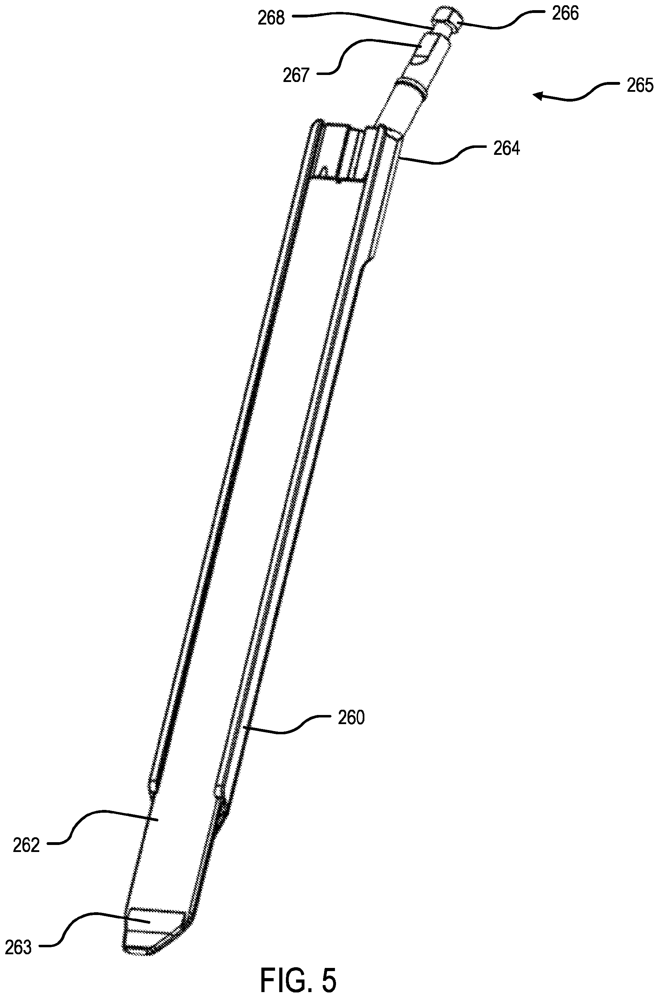

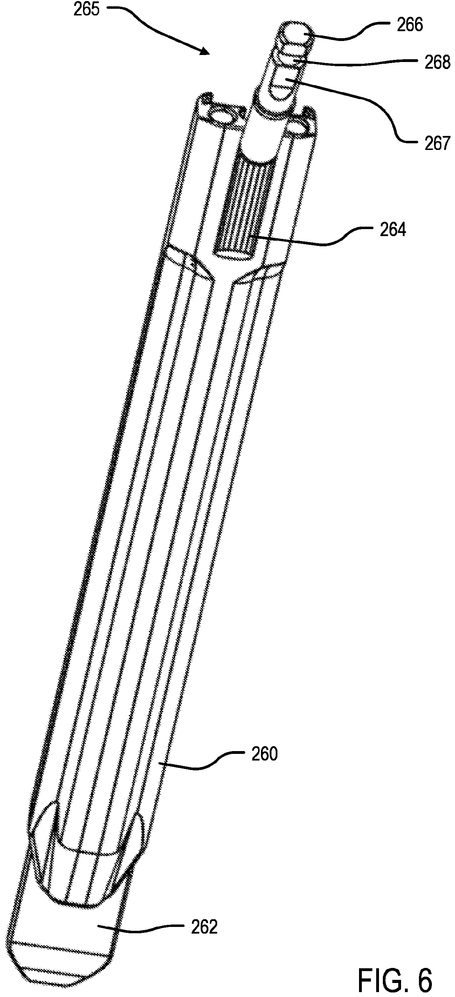

Referring generally to FIGS. 5-11, various views of a modular blade 260 and an extendable blade 262 having a footed tip 263, a quick connect handle 270, and a retractor mount coupler 280 are disclosed. FIG. 5 is a perspective view of the inside surfaces of the modular blade 260 and extendable blade 262 and FIG. 6 is a perspective view of the outside surfaces of the modular blade 260 and extendable blade 262. In the example embodiment, the extendable blade 262 has a footed tip 263 and is slidably coupled to the modular blade 260 similarly as explained above. Accordingly, duplicative description is omitted or only briefly described again. In this embodiment, modular blade 260 comprises an attachment rail 264 on the outside surface thereof adjacent the proximal end. Attachment rail 264 may include various surface texturing on the outside surface thereof, e.g., rail like peaks and channel like valleys therebetween extending in a proximal to distal direction around the outside curved surface of attachment rail 264. In at least some embodiments, attachment rail 264 is integrally formed with modular blade 260 and in others it may be removably coupled thereto. In various embodiments, attachment rail 264 may include attachment shaft 265 extending from the proximal end of modular blade 260, for example. The proximal most portion of attachment shaft 265 may comprise a generally cylindrical extension shaft having a planar indent 267, a necked down portion 268, and an end 266 that is wider than the necked down portion 268, for example. In various embodiments, the attachment shaft 265 may quickly couple to and uncouple from a quick connect handle 270, for example.

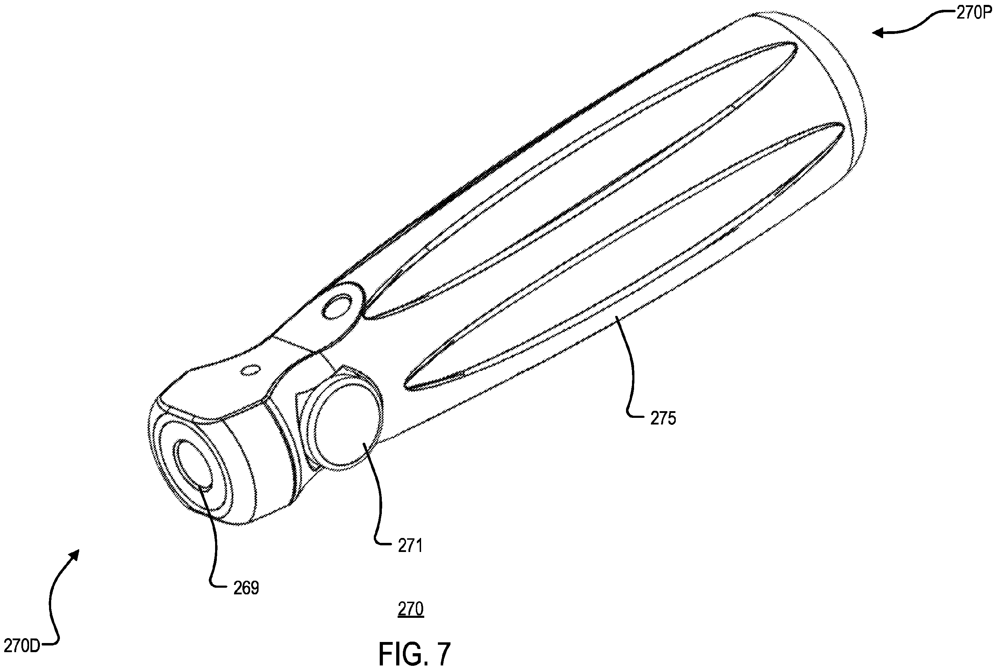

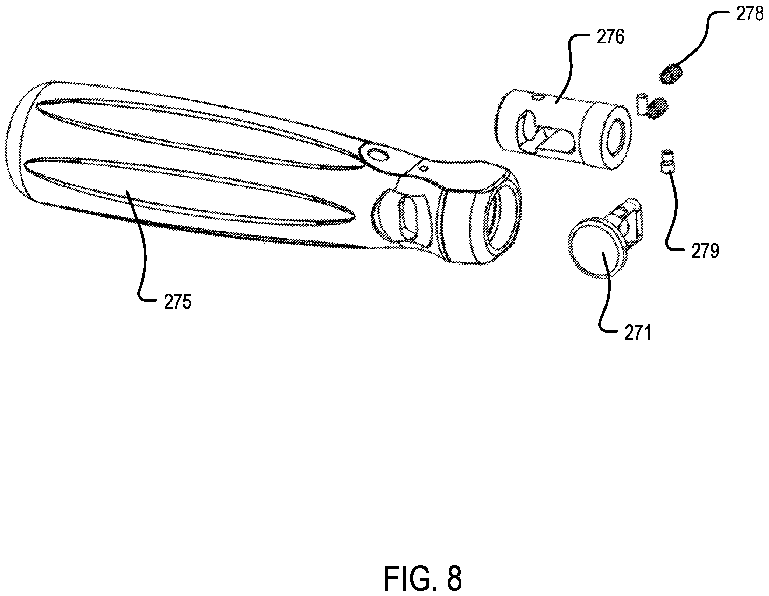

FIG. 7 is a perspective view of a quick connect handle 270 and FIG. 8 is an exploded parts view of the quick connect handle 270. In the example embodiment, quick connect handle 270 extends in a longitudinal direction from a proximal end 270P to a distal end 270D. The distal end 270D may comprise a coupling aperture 269 for connecting to an attachment shaft 265 of a modular blade 260 when attachment shaft 265 is inserted therein, for example. Quick connect handle 270 may include a main body portion 275 or handle and the distal end 270D may comprise a coupling mechanism having various mating features comprising a size and shape generally corresponding to a size and shape attachment shaft 265, for example. In the example embodiment, the pin 272 and mating features 274 are actuated by actuator 271. Quick connect handle 270 may couple to modular blade 260 by depressing actuator 271 and sliding attachment shaft 265 into aperture 269 such that sliding barrel 276 lockingly engages attachment shaft 265, for example. In various embodiments, barrel 276 may be biased towards the proximal end 270D by set pins 279 and springs 278. In various embodiments, upon activation of actuator 271, e.g., by depressing actuator 271 button, barrel 276 may linearly translate forward to securely couple to modular blade 260.

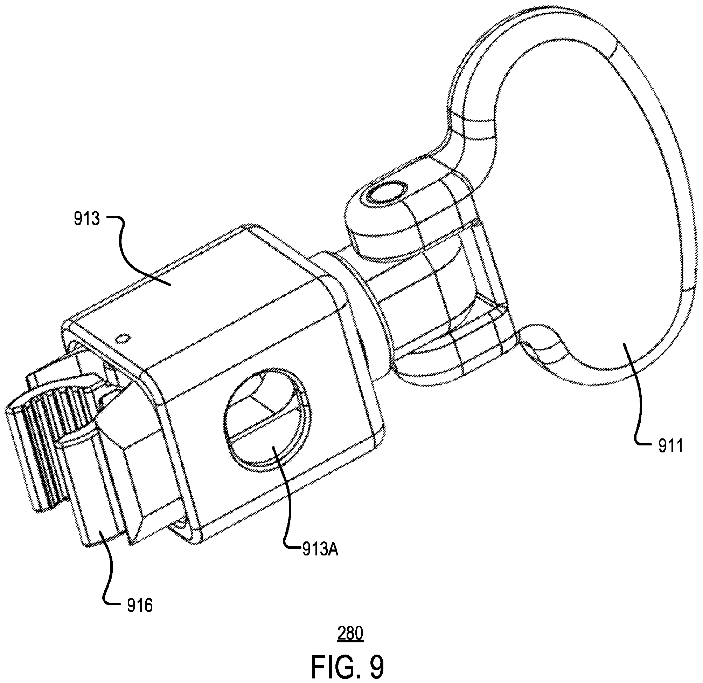

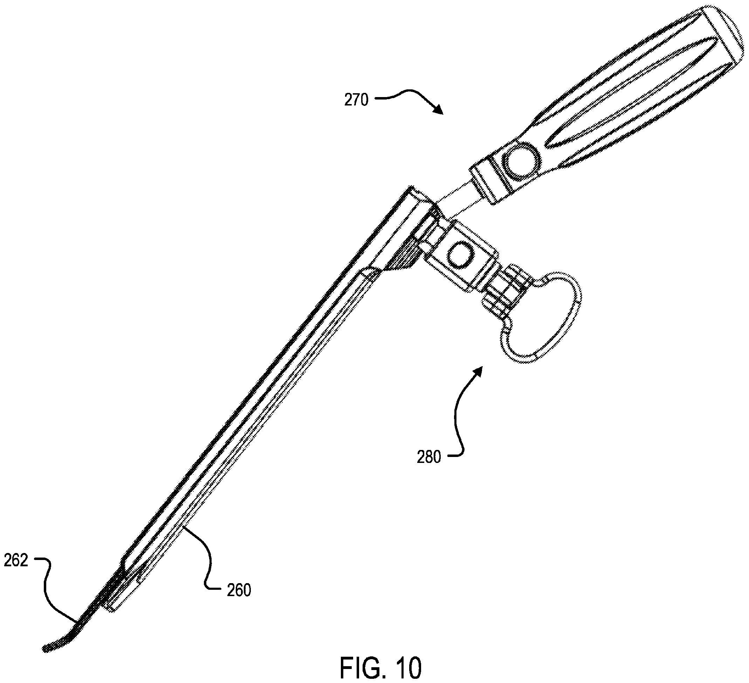

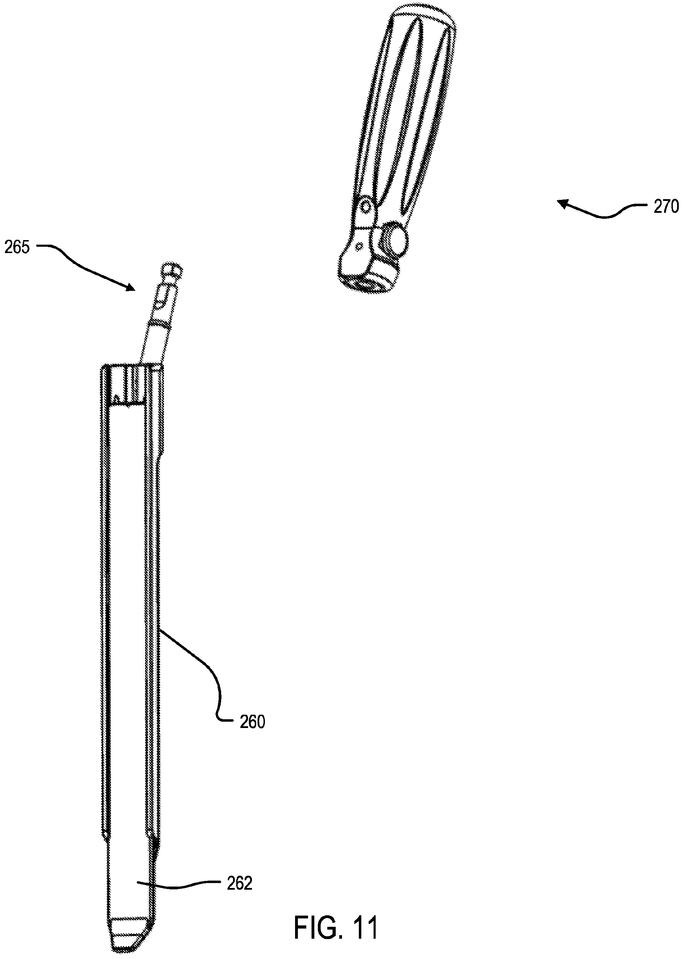

FIG. 9 is a perspective view of a retractor mount coupler 280. In this embodiment, retractor mount coupler 280 may include a pair of gripping arms 916 for gripping on to the outside textured surface of rail 264, for example. Additionally, retractor mount coupler 280 may include a lever 911 and a body 913 having an aperture 913A. Lever 911 may function in the same or substantially the same way as previously explained with respect to free hand module 900. As seen best in FIG. 129, retractor mount coupler 280 may couple and couple to a rod, pole, table mount extension, and/or lateral arm 1201 of a secondary module 1200, for example. FIG. 10 is a perspective view of the modular blade 260 and extendable blade 262 of FIGS. 5-6 coupled to the quick connect handle 270 of FIGS. 7-8 and the retractor mount coupler 280 of FIG. 9. FIG. 11 is a perspective view showing the quick connect handle 270 uncoupled from the attachment shaft 265 of modular blade

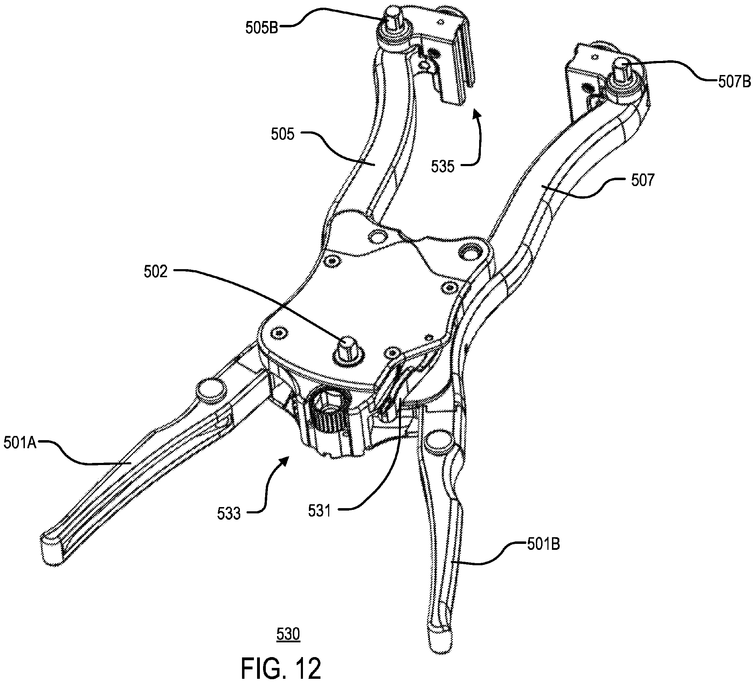

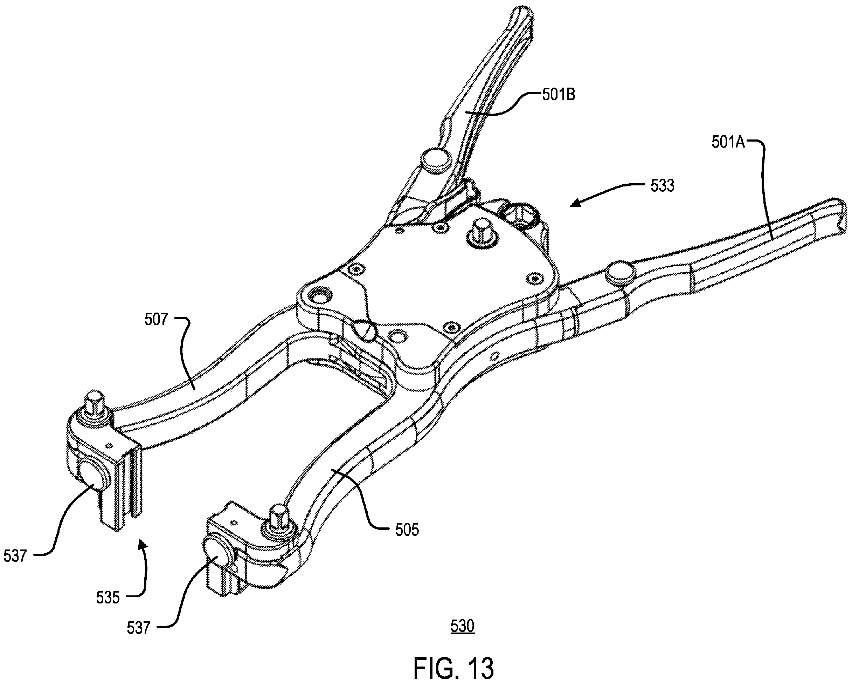

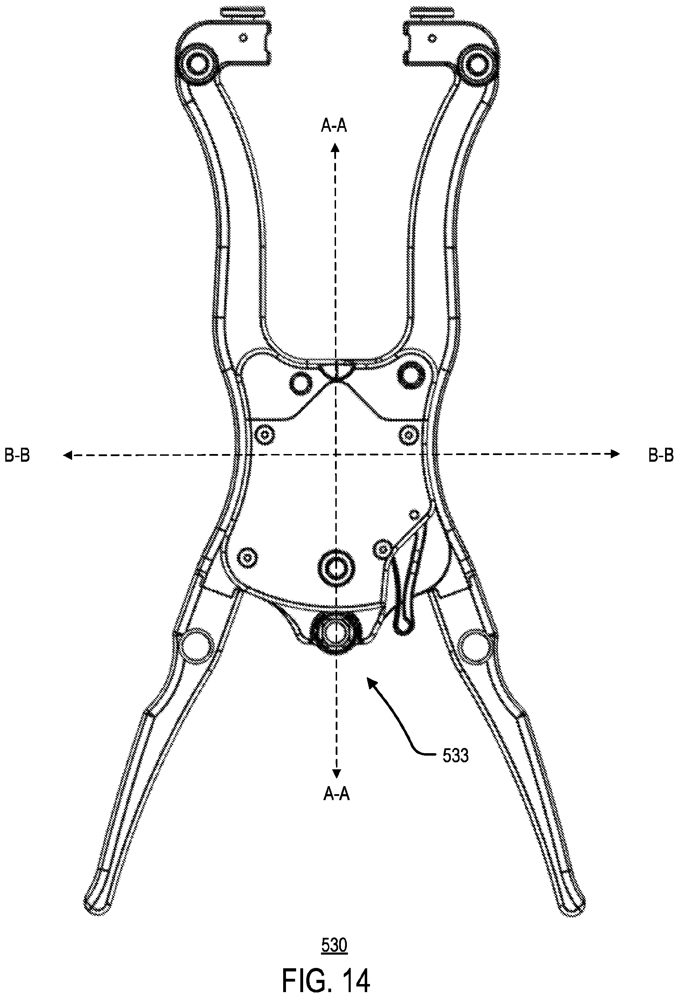

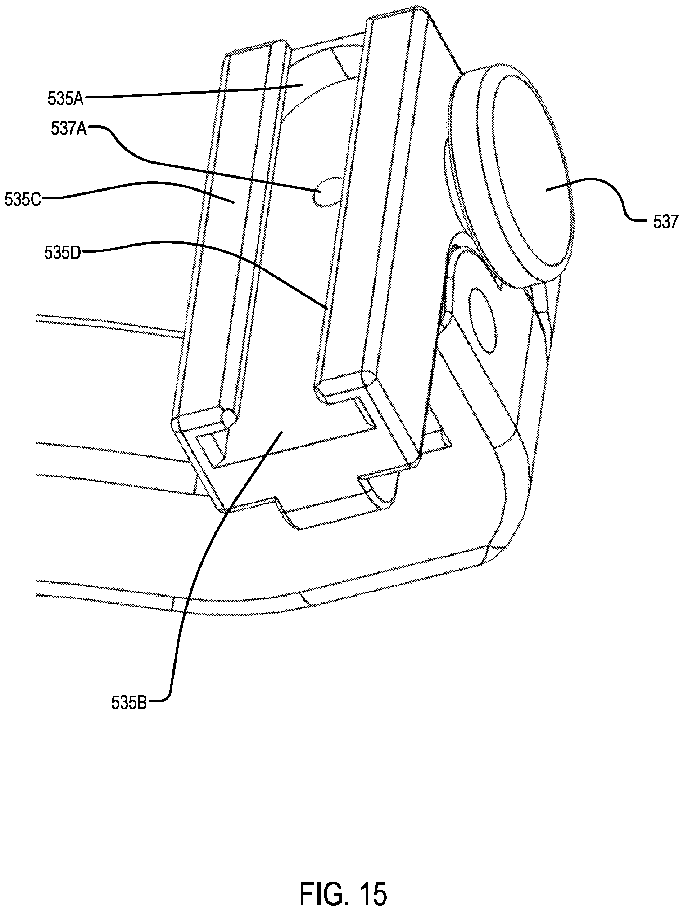

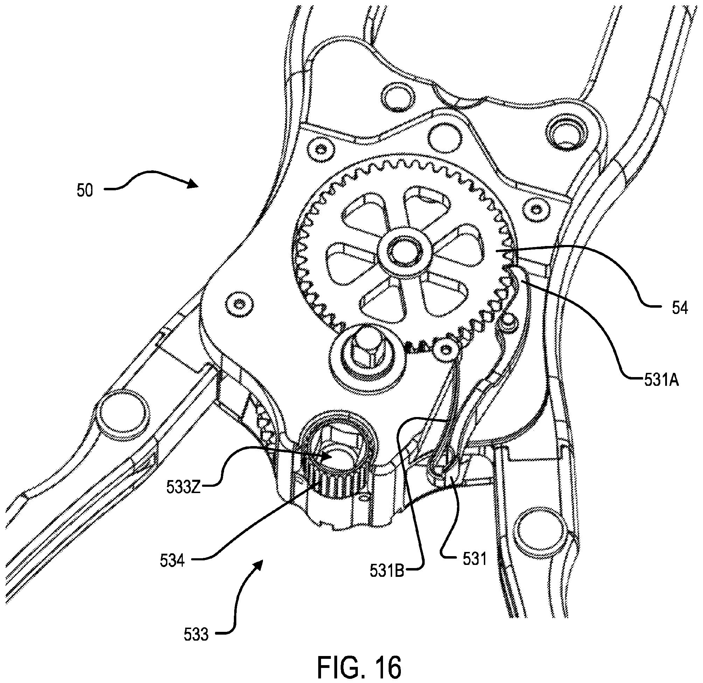

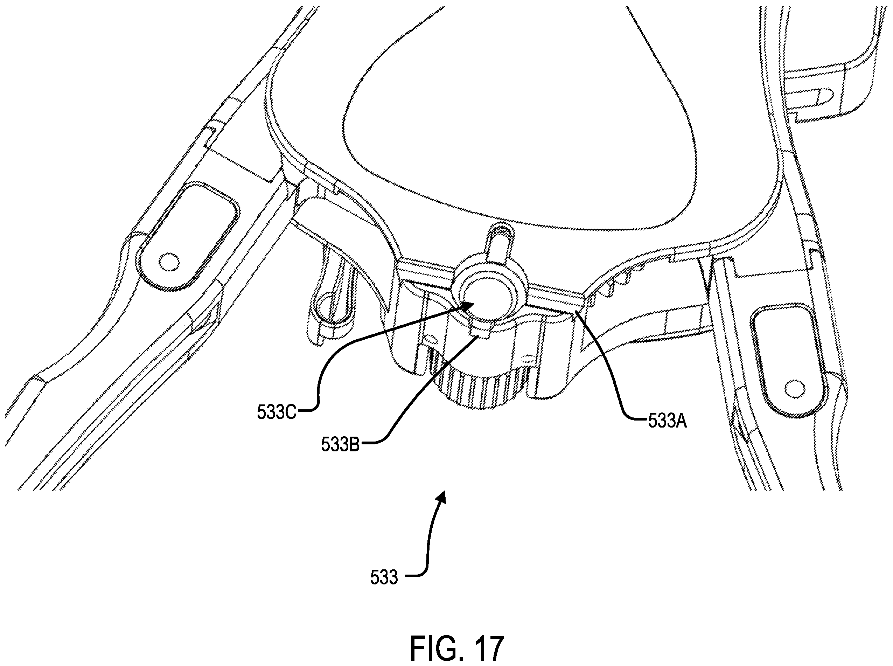

Referring generally to FIGS. 12-17 an additional embodiment of a modular retractor 530 is disclosed. FIGS. 13 and 14 are various perspective views of modular retractor 530. FIG. 14 is a top down view of modular retractor 530 and FIG. 15 is a bottom perspective view of a blade attachment mechanism 535. FIG. 16 is an enlarged view of the embodiment of FIGS. 13-15 with the top cover removed for ease of understanding of the internal gear system. FIG. 17 is an enlarged view of the embodiment of FIGS. 13-15 from a bottom perspective showing various structural features of a table mount quick release coupler 533.

In the example embodiment, modular retractor 530 may include handles 501a, 501b and arms 505 and 507. The handles and/or actuator 502 may serve to open the retractor 530 by spreading the arms 505, 507 apart from one another as explained previously. Modular retractor 530 may differ from retractor 500 in a few ways. For example, the blade attachment mechanism 535 may be configured for bottom loading rather than top loading, the blade release actuator 537 may be disposed on a side surface of blade attachment mechanism 535 rather than a top surface, a release mechanism 531 may be relied upon rather than pawl 504, and a table mount quick release connection 533 may be provided rather than table mount arm 506, for example.

As seen best in FIGS. 13 and 15, blade attachment mechanism 535 may be configured for bottom loading of various blade engagement features, for example blade engagement feature 146 shown in FIGS. 108B, 108C, and 108D. Generally, blade attachment mechanism 535 may have a size and shape generally corresponding to a size and shape of a corresponding blade engagement feature 146. In the example embodiment, blade attachment mechanism 535 includes a channel 535B that is open to the bottom and closed at the top by a curved top surface 535A, for example. The curved top surface may have a curvature generally corresponding to a curvature of the curved uppermost surface 146A and the channel 535B may have a width, length, and height generally corresponding to a width, length, and height of raised rail portion 146B, for example. In some embodiments, the curved top surface 535A may be referred to as a stop surface and/or stopping wall. In various embodiments, channel 535B may be flanked by supports 535C, for example. In use, when blade engagement feature 146 is securely coupled to blade attachment mechanism 535 the side surfaces of platform 146D may contact the side surfaces 535D of supports 535C such that the channel portions 146C are mated with supports 535C, for example. Additionally, in various embodiments the side surfaces 535D may be chamfered and/or inclined, for example as seen best in FIG. 15. This arrangement may facilitate insertion of a blade thereon, for example. Furthermore, in various embodiments an outside surface of a corresponding blade and/or platform 146D may contact the outermost surface of supports 535C. Further still, blade release mechanism 537 may securely couple and uncouple a blade to blade attachment mechanism 535 when actuated by moving a spherical detent 537A in and out of a corresponding aperture of a blade.

As seen best in FIG. 16, modular retractor 530 may include a distraction mechanism 50. In this embodiment, spur gear 54 includes a plurality of teeth on a side surface thereof but may not include a plurality of teeth on a top surface thereof like the example modular retractor 500 embodiment. Additionally, a release mechanism 531 may include a tooth 531A or tip at an end thereof having a size and shape generally corresponding to a valley between adjacent teeth of spur gear 54. Release mechanism 531 may be biased by a spring tab or leaf spring 531B which naturally urges tooth 531A into a meshed arrangement with the teeth of spur gear 54 such that the spur gear 54 is prevented from rotating in a direction which would cause the arms of modular retractor 530 to collapse or close. Additionally, because release mechanism 531 may pivot in and out of a meshed arrangement with spur gear 54 an end user may cause expansion or distraction between arms 505, 507 of modular retractor 530 without needing to actuate release mechanism 531. In this way, release mechanism 531 functions similarly to a pawl preventing the collapse of arms 505, 507 while simultaneously allowing, for example, an uninhibited expansion of arms 505, 507.

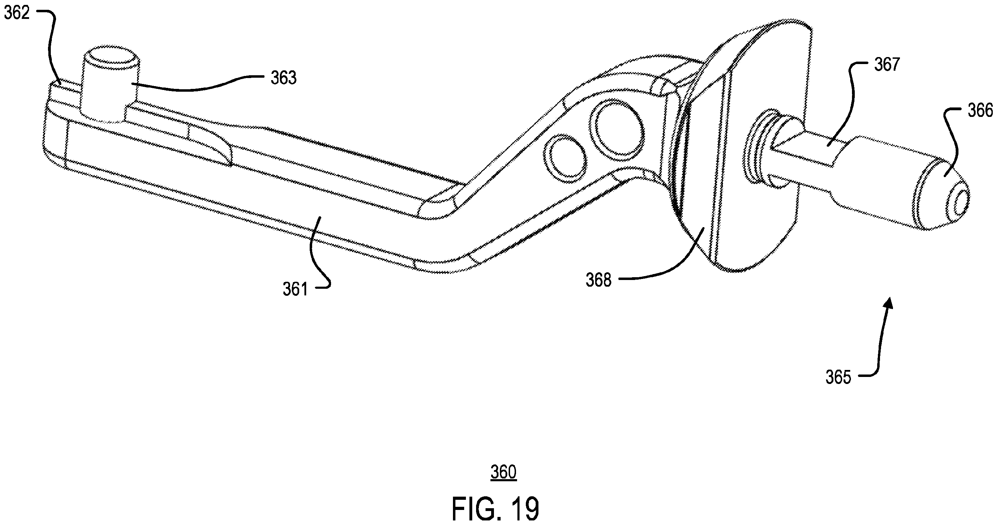

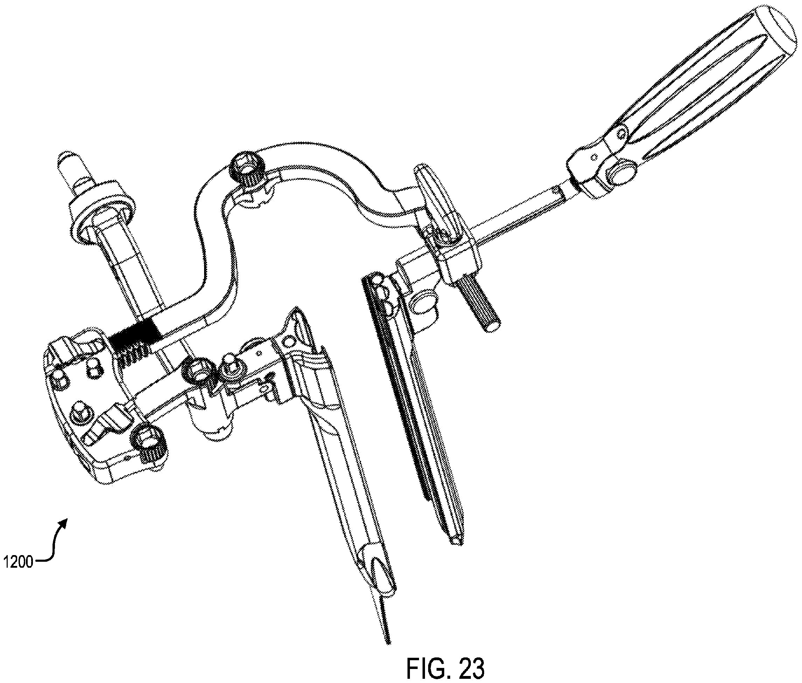

As also seen best in FIGS. 16 and 17, modular retractor 530 may include a quick connect table mount 533. Quick connect table mount 533 may include an aperture 533Z and a tightening knob 534. Aperture 533Z may have a size and shape corresponding to a square or polygonal driver, for example a drive end of a wrench such as the egg wrench 10 illustrated in FIG. 23. As seen best in FIG. 14, quick connect table mount 533 may be generally disposed in a central position of the main retractor body and when viewed in a plan view may be aligned along a longitudinal axis bisecting the retractor body. This arrangement may facilitate a symmetrical load distribution, for example. However, it shall be understood that quick connect table mount 533 may be alternately disposed, for example on a side surface on the left side, medial or central area, and/or right side of the retractor body (with respect to plan view of FIG. 14). Additionally, a plurality of quick connect table mounts 533 may be disposed in any viable region of modular retractor 530 and the various add on modules disclosed herein. As seen best in FIG. 17, quick connect table mount 533 may be configured to receive a corresponding post 363 and/or rail 362 of a quick connect arm 360 (see FIGS. 19-20). In the example embodiment, quick connect table mount 533 may include a centrally disposed mating aperture 533C accessible from a bottom side of modular retractor 530, for example. In some embodiments, mating aperture 533C is coaxially aligned with aperture 533Z although this is not a requirement. In various embodiments, mating aperture 533C may include a circumferential ring surface having a relatively greater diameter than the central portion of aperture 533C to facilitate seating of post 363 (see FIGS. 19-20) in an arrangement similar to concentric circles of varying depths. Additionally, various counter torque mating features may be disposed around and/or surround aperture 533C, for example. In the example embodiment, a first groove 533A extends in a direction that is substantially parallel with the longitudinal axis of modular retractor 530 and a second groove 533B extends in a direction that is substantially perpendicular with the longitudinal axis of modular retractor 530, e.g. second groove 533B extends in a lateral direction with respect to modular retractor 530. In the example embodiment, the first and second grooves 533A, 533B may resemble a cross shape and/or an X shape. Additionally, the ends of grooves 533A, 533B may be open or closed, for example one side of groove 533B is closed and has an arcuate end surface which ensures proper alignment quick connect arm 360, for example. In various embodiments, the rail 363 of quick connect arm 360 may nest within at least one of grooves 533A, 533B. Accordingly, in this embodiment an orientation of quick connect arm 360 is adjustable between a direct head on orientation type and a side or lateral orientation type, for example. At least one advantage of this configuration may be providing flexibility in orientation to a surgeon depending on different types of procedures being performed and or changes in orientation mid-procedure.

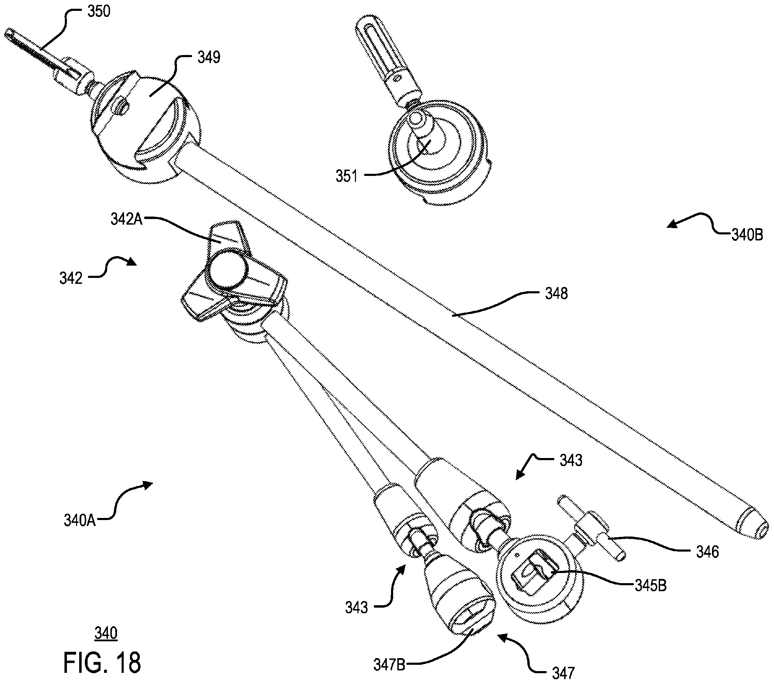

FIG. 18 is a perspective view of a table mount system 340 adapted for use with various retractor components disclosed herein. For example, various armatures of a quick connect table mount system may be used for supporting and manipulating various retractor embodiments disclosed herein. In the example embodiment, table mount system 340 may include a first armature 340A and a second armature 340B. The first armature 340A may be slidably connected to the second armature 340B by armature connection mechanism 345. In at least one embodiment, second armature 340B may include a table mount channel 349 and a table mount clamp 350. In use the second armature 340B may be rigidly and removably secured to an operating table by tightening clamp 350 such that table mount channel 349 is tightened to the table and arm 348 extends in a vertical direction with respect to a horizontal surface or plane of a table (not illustrated). Thereafter, the first armature 340A may be coupled to arm 348 by positioning arm 348 within the aperture of armature connection mechanism 345 and tightening turnkey 346 such that a movable platform 345B clamps down on to armature 348 by closing and/or reducing the size of the aperture, for example. In the example embodiment, movable platform 345B has a channel for seating the curved surfaces of arm 348 and in some embodiments may have grooving or other texturing to facilitate a relatively strong connection.

First armature portion 340A may include a first arm 341A and a second arm 341B that are hingedly connected together by hinge mechanism 342, for example. In various embodiments, hinge mechanism 342 may allow for a full 360 degree rotation, or a subset thereof. At least one embodiment may include corresponding teeth that may mesh together when tightened or clamped together by a tightening knob 342A that urges the corresponding teeth into corresponding valleys, for example. In various embodiments, second arm 341B may be movably coupled to armature connection mechanism 345 by a ball and socket joint 343, for example. Additionally, first armature 341 may be coupled to a snap on connector 347 by a ball and socket joint 343, for example. Consistent with the disclosure herein, it shall be understood that any connection between the various armatures of disclosed table mount systems may be a rotatable hinge like connection, a sliding connection, and/or a ball and joint connection. Additionally, these connection types may be readily swapped and our substituted. For example, post 351 may be inset within a hollow interior of any armature end to change the connection type and/or functionality depending solely on the particular needs of an end user.

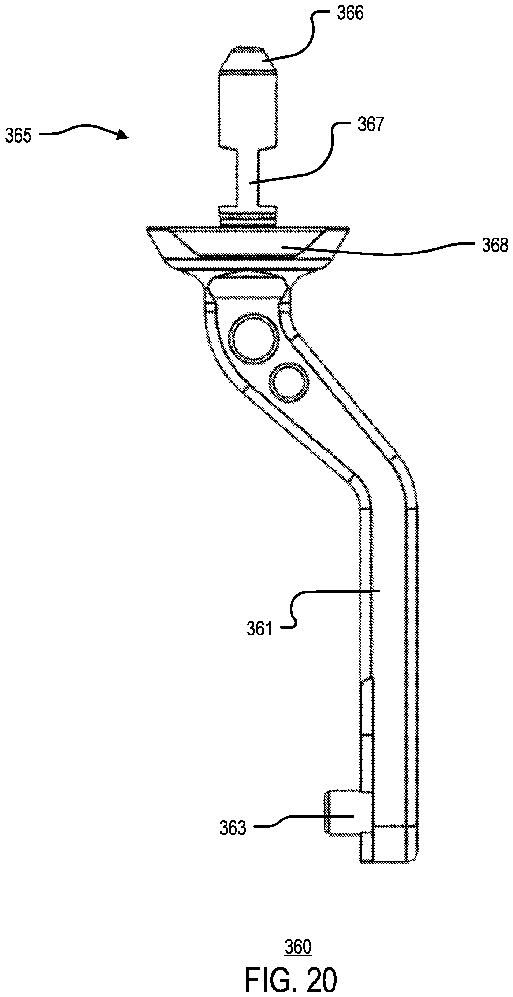

FIG. 19 is a first perspective view of a quick connect coupler 360 for connecting various retractor embodiments to various quick connect table mount systems disclosed herein. FIG. 20 is a side view of the quick connect coupler 360. Quick connect coupler 360 may include an arm 361 supporting a post 363 and rail 362 on a first end thereof. In various embodiments, the arm 361 may follow a diagonal, straight, and/or curved profile, for example. On an opposite end, quick connect coupler 360 may include an armature coupler 365, for example. Armature coupler 365 may include a post having an inclined, chamfered, and/or dimpled end 366, for example. Additionally, armature coupler 365 may include a grooved portion 367 to facilitate a rigid and secure engagement with a quick connect coupler of a table mount system, for example connector 347 shown in FIG. 18. Furthermore, a base portion of quick connect coupler 360 may include a counter torque surface 368 for resisting a rotation of quick connect coupler 360, for example surface 368 may directly contact a corresponding counter torque surface 347B of connector 347 (see FIG. 18). It shall be appreciated that armature coupler 365 may take any shape and have any form and type of various indentations, outdents, apertures, posts, slots, and etc. to facilitate attachment to a table mount arm whether in a snap on quick connect style as illustrated in the corresponding FIGS. or by, for example, a clamp on ratcheting style or even a mushroom expansion style.

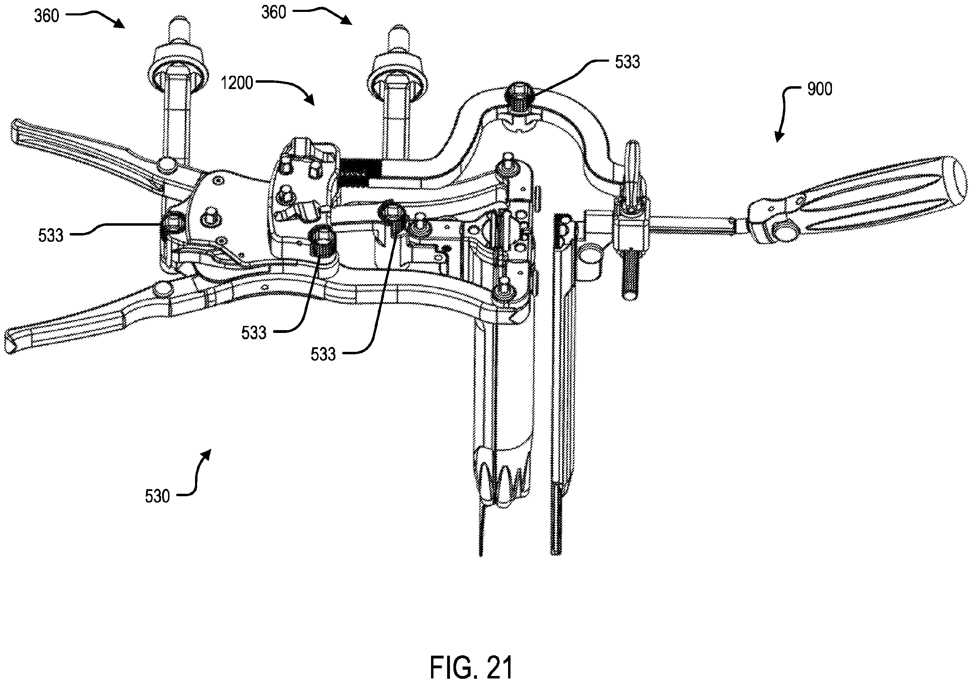

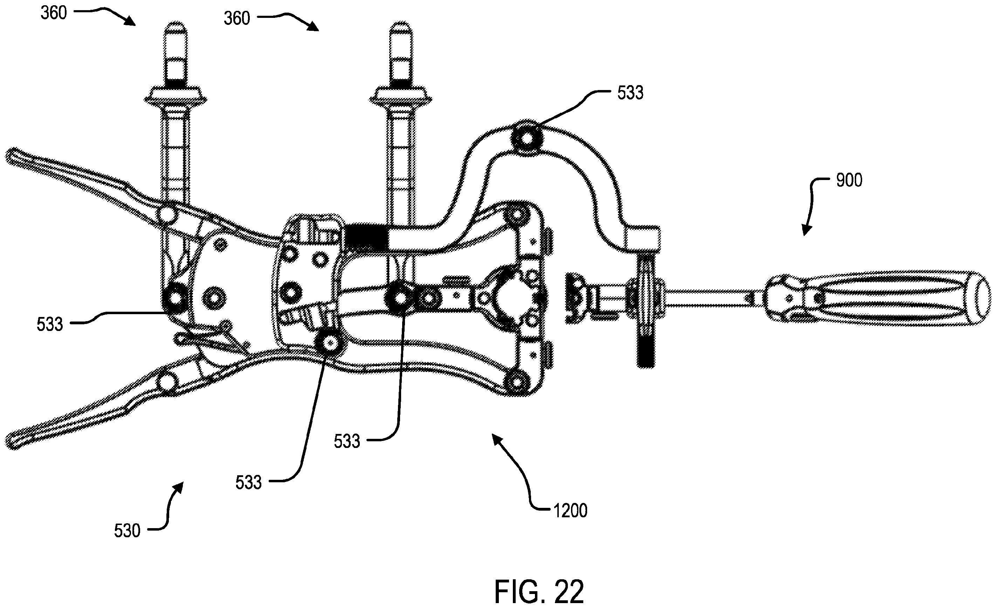

FIG. 21 is a perspective view of a modular retractor system including the quick connect couplers of FIGS. 19-20. FIG. 22 is a top down view of the system of FIG. 21. Consistent with the disclosure herein, modular retractor 530 is securely coupled to a secondary module, e.g., module 1200. Module 1200 may have the same, similar, and/or substantially the same features and functionality as the various other secondary modules discussed above. However, in this embodiment, secondary module 1200 is capable of linearly extending a centrally disposed first arm and a C shaped second arm, for example. In this embodiment, the C shaped second arm is supporting a free hand module 900 and the first arm is securely connected to a quick connect arm 360 via a table mount quick release coupler 533, for example. Additionally, a body portion of module 1200 includes a table mount quick release coupler 533 and the second C shaped arm includes a table mount quick release coupler 533. Additionally, it is shown that modular retractor 530 is securely connected to a quick connect coupler 360 at the table mount quick release coupler 533. Accordingly, various modular retractor systems may comprise a plurality of quick release couplers 533 whether they be on the primary retractor 530 or secondary module 1200, for example. In this way, a surgeon has maximum flexibility in attaching the modular retractor system to a table mount. FIG. 128 is a perspective view of module 1200 in an uncoupled position with respect to modular retractor 530. FIG. 129 is a perspective view of module 1200 coupled to modular retractor 530. In the example embodiment, retractor mount coupler 280 is secured to attachment rail 264 of the modular blade 260 and extendable blade 262. Additionally, the retractor mount coupler 280 is coupled to lateral arm 1201 of retractor module 1200.

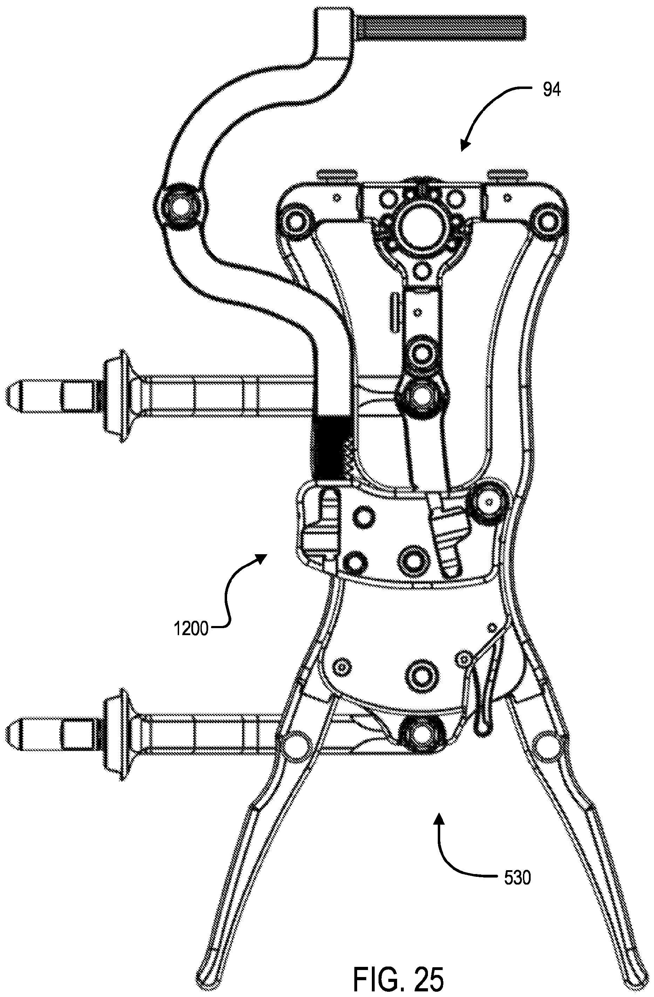

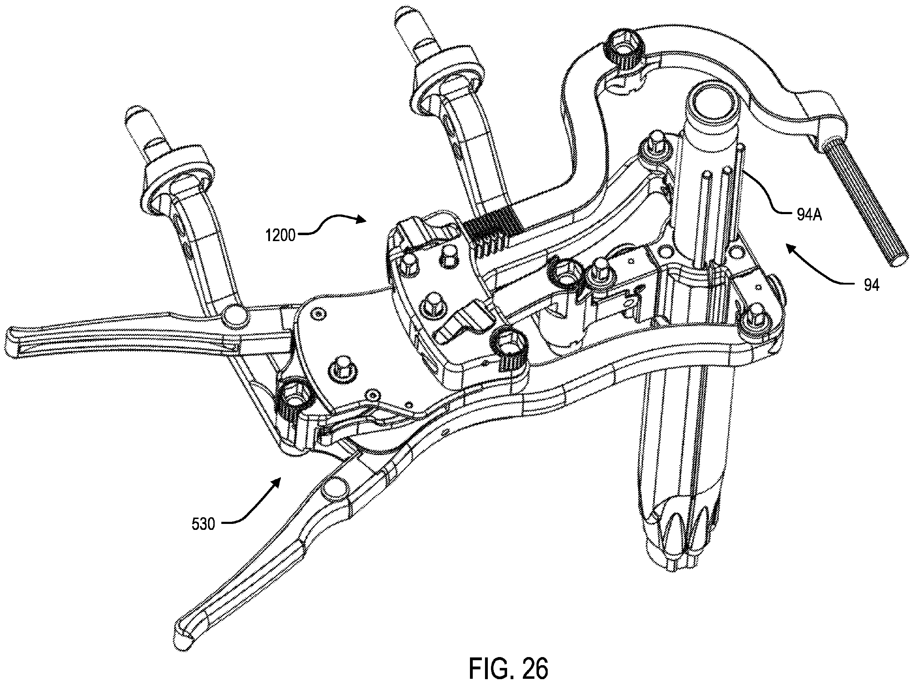

FIG. 25 is a top down view of modular retractor 530 supporting first and second blades and module 1200 supporting a third blade. FIG. 26 is a perspective view of three blades being slidably coupled to a dilator 94. An example method of use will be disclosed. In a first step, a surgeon may insert an initial dilator or pin, e.g., innermost dilator. In some embodiments, and depending on the particular surgical approach innermost dilator may be inserted into a patient from a lateral, anterior, or trans psoas approach, e.g. Once the initial dilator is inserted in the patient, a second dilator having a relatively wider size may be insert over the initial dilator. After second dilator is slipped over innermost dilator, a third dilator having a relatively wider size than second dilator may be slipped over second dilator. Any number of successive and iteratively increasing in size dilators may be slipped over one another in this process. Thereafter, an outermost dilator 94 having a plurality of rail portions 94A may be slipped over the immediately prior dilator, e.g. third dilator.

Next, a surgeon may position modular retractor 530 and retractor module 1200 over the outermost dilator 94. For example, a surgeon may install first and second blades to the first and second arms of modular retractor 530 and a third blade may be installed on the proximal arm of retractor module 1200. The three blades may be collapsed such that edge portions contact one another and a circular void space is formed by the interior surfaces of the three blades. Next, the surgeon may slip the three blades over the outermost dilator 94 such that the corresponding channel portions of the blades slidably couples to the rail portions 94A of the outermost dilator. Thereafter, the surgeon may move the modular retractor 530 and retractor module 1200 such that the blades slide down along the length of outermost dilator and into the operative corridor. Once the surgeon has moved the three blades into the operative corridor, and the blades are supporting adjacent tissue, the surgeon may remove the outermost dilator 94. After the outermost dilator 94 has been removed, the surgeon can freely manipulate any one of the three blades in any manner or relative movement as previously explained to enlarge the operative corridor.

### Additional Retractor Embodiments:

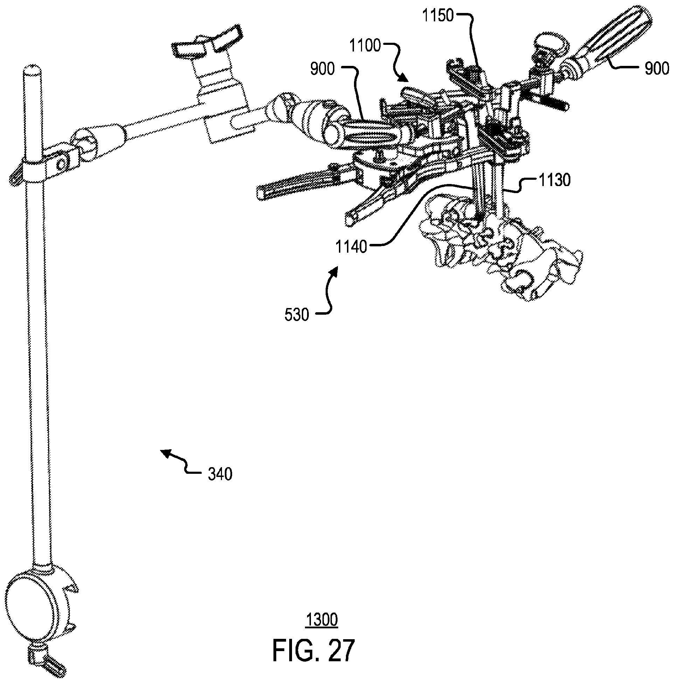

Referring generally to FIGS. 27-36 an example modular retractor system including a modular retractor 530, a fifth module 1100, first and second freehand modules 900, a cannula 1130, a blade 1140, and a support module 1150 are disclosed. In various embodiments, modular retractor 530, and fifth module 1100 may include the same, substantially the same, and/or similar components and functionality as the above disclosed retractor embodiments. Accordingly, those with skill in the art will understand the general principles, modes of operation, and associated methods of each example embodiment may be combined and/or modified in view of the skill of a person of ordinary skill in the art. The embodiments and methods shown in FIGS. 27-36 may be adapted for a cervical tissue retraction procedure and/or for performing a distraction of a disc space in the cervical portion of the spine, e.g., by spreading apart adjacent vertebrae and/or adjusting an alignment of the adjacent vertebrae. It shall be understood that the embodiments and methods are not limited to applicability of the cervical portion of the spine and may also be applied to other portions of human anatomy, e.g., the thoracic and/or lumbar areas of the spine and even other boney anatomy not associated with the spine.

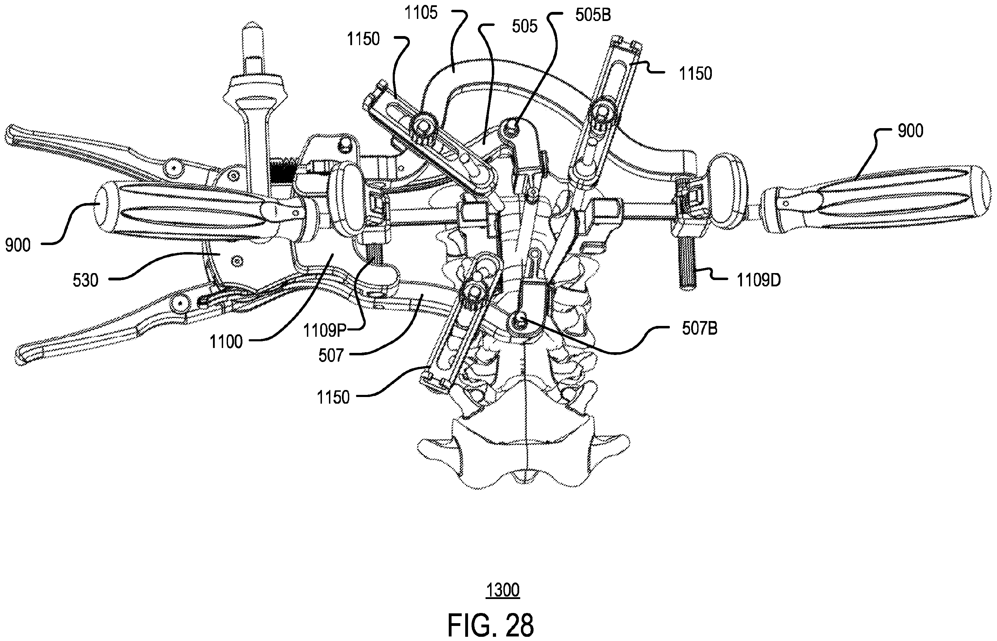

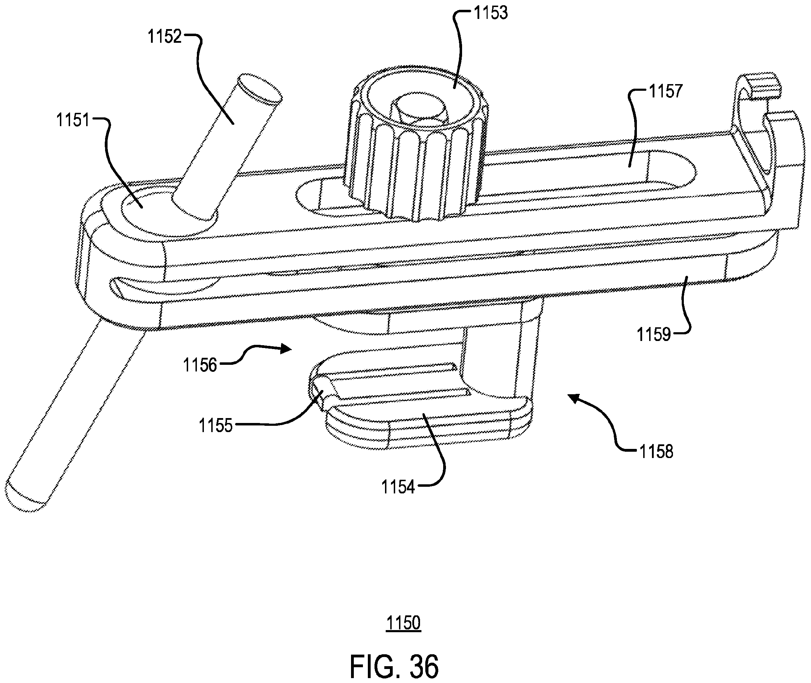

FIGS. 27-30 are perspective views of a cervical modular retractor system 1300 during use. In the example embodiment, modular retractor 530 is securely connected to fifth module 1100 in the same, similar, or substantially the same way as disclosed above. Table mount system 340 may be securely coupled to and uncoupled from modular retractor 530 and/or fifth module 1100 at various attachment locations similarly as explained above. FIG. 28 illustrates a top down view of modular retractor system 1300 with the table mount system 340 removed for ease of explanation. As illustrated, a plurality of support modules 1150 are removably coupled to modular retractor 530 and fifth module 1100. A first support module 1150 is attached to arm 507 of modular retractor 530; and a second support module 1150 and third support module 1150 are attached to arm 1105 of fifth module. As explained in further detail below in conjunction with FIG 36, support modules 1150 may be used to support a lighting system, an endoscope system, and/or a camera system.

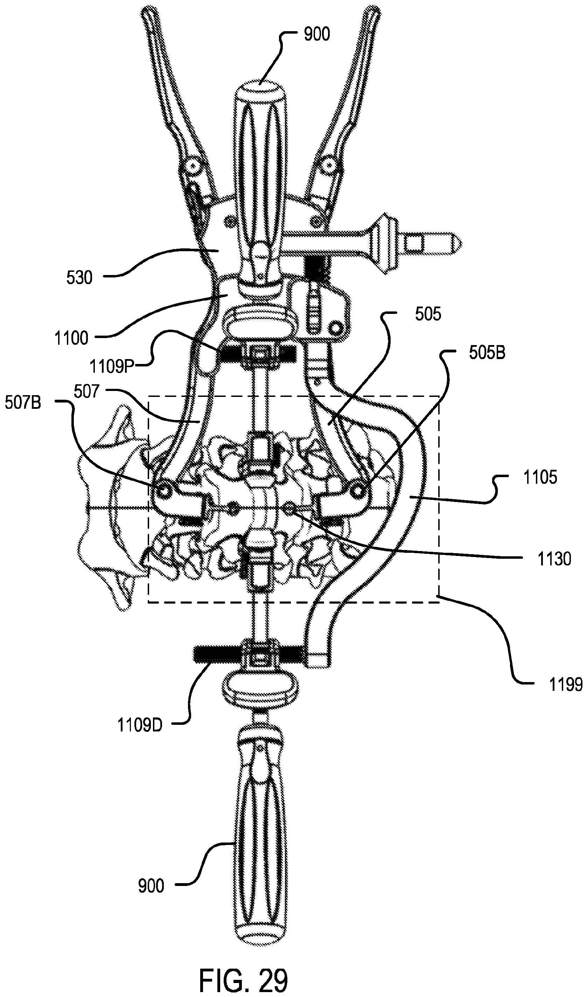

FIG. 29 is a top down view of a modular retractor system 1300 with the support modules 1150 removed for ease of understanding. As illustrated, the surgical approach is from a lateral perspective towards a cervical portion of the spine. In various embodiments, a first pin delivery cannula 1130 may be removably coupled to arm 505 and a second pin delivery cannula 1130 is removably coupled to arm 507 of modular retractor 530. A first free hand module 900 is secured to distal module mount 1109D and a second free hand module 900 is secured to proximal module mount 1109P, for example. A cervical blade 1140 is releasably coupled to each of the free hand modules 900, for example.

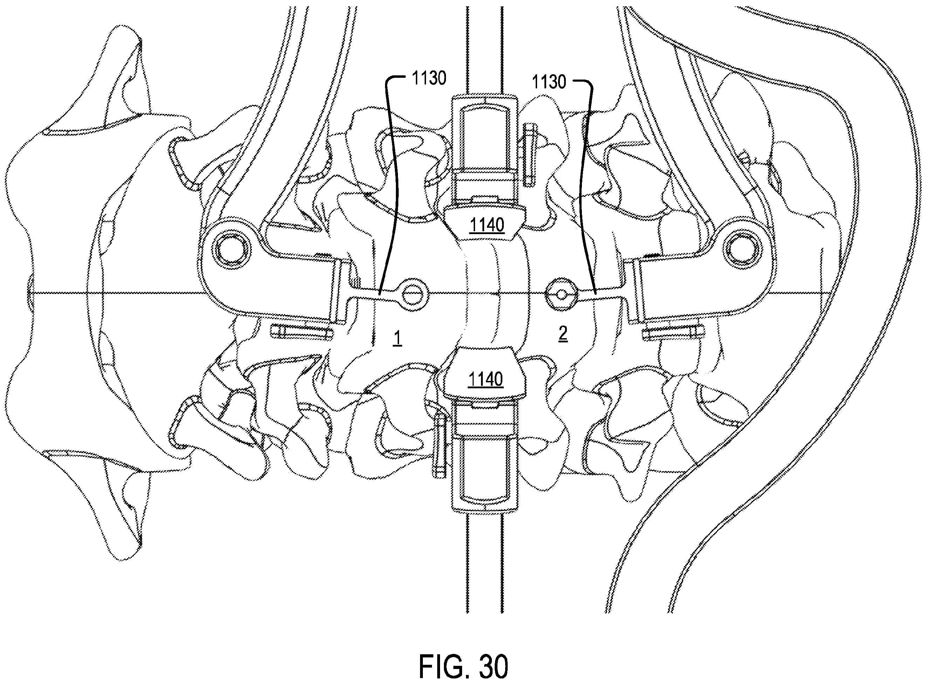

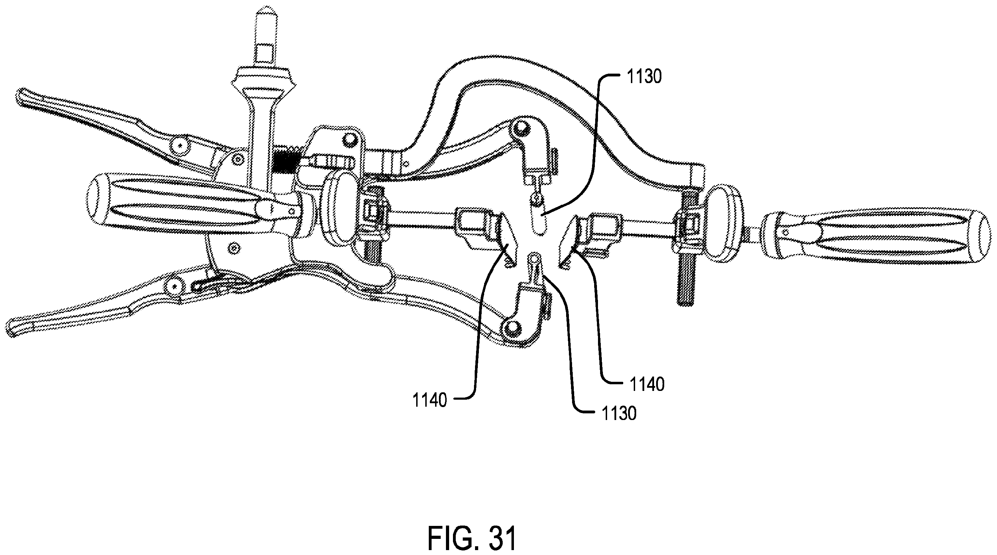

FIG. 30 is an enlarged top down view of region 1199 of FIG. 129. In the example embodiment, the first pin delivery cannula 1130 is positioned above a first vertebrae 1 and the second pin delivery cannula 1130 is positioned above a second vertebrae 2 adjacent to the first vertebrae 1. In this embodiment, the first and second pin delivery cannulas 1130 are generally aligned with a centerline of a caudal to cephalad extension direction of the spine (see FIGS. 142, 143). The first cervical blade 1140 and second cervical blade 1140 are positioned on opposite lateral sides of the first vertebrae 1 and second vertebrae 2 at a position that approximates the disc space between the first vertebrae 1 and second vertebrae 2, for example. FIG. 31 illustrates the configuration of FIG. 30 with the cervical portion of the spine removed for ease of visualizing the relative location of the pin delivery cannulas 1130 with respect to the cervical blades 1140.

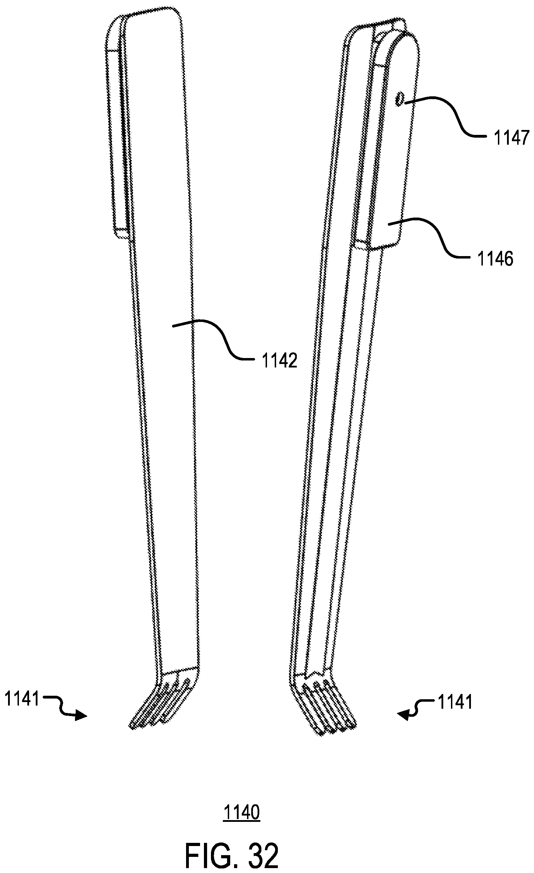

FIG. 32 is a perspective view of a pair of cervical blades for use with various modular retractor systems disclosed herein. In the example embodiment, cervical blade 1140 may be a relatively thin and low profile blade adapted for use in the cervical area of the spine, for example. A proximal end of cervical blade 1140 may include an engagement feature 1146 having the same, similar, or substantially the same functionality as explained above. Blade 1140 may have a substantially planar inside surface 1142 having a width that generally tapers from the proximal end to the distal end, for example. Additionally, the distal end may include a footed tip 1141 that is angled with respect to the planar inside surface 1142. In various embodiments, the footed tip 1141 may include various prongs with gaps therebetween or it may be substantially solid. In this embodiment, engagement feature 1146 is configured for bottom loading in a blade coupling portion 535 shown in FIG. 15. In use, blade 1140 may securely couple to blade coupling portion 535 by sliding blade 1140 into channel 535B while detent 537A engages with detent receiving aperture 1147 (see FIG. 15), for example. To remove blade 1140, an end user may actuate the blade release mechanism which will pull detent 537A out of the detent receiving aperture 1147 such that the blade 1140 may be slipped out.

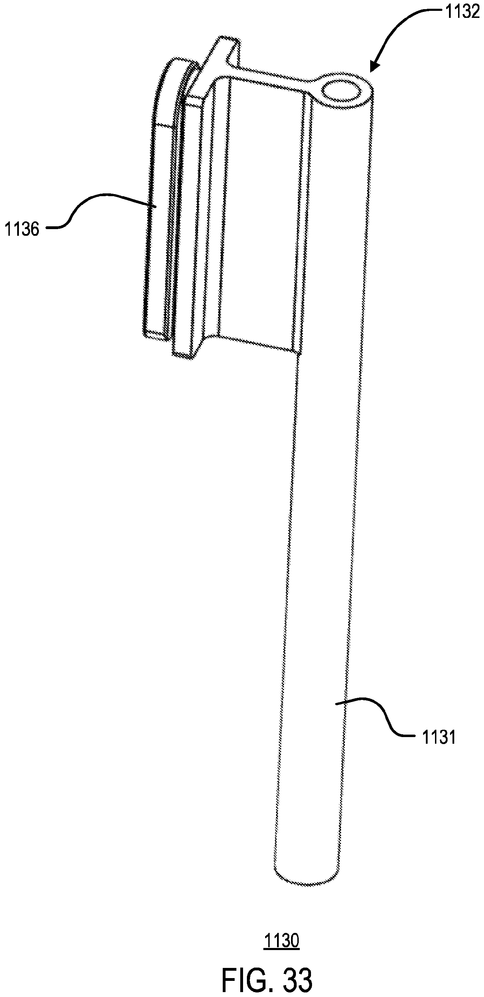

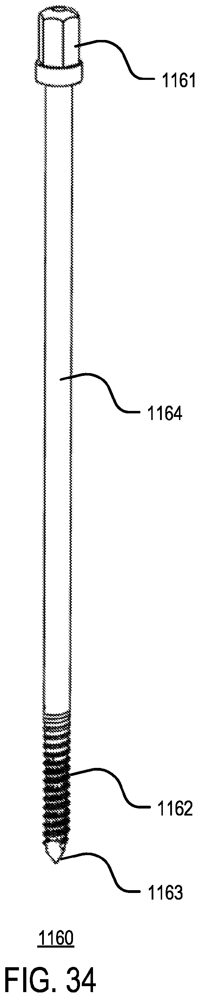

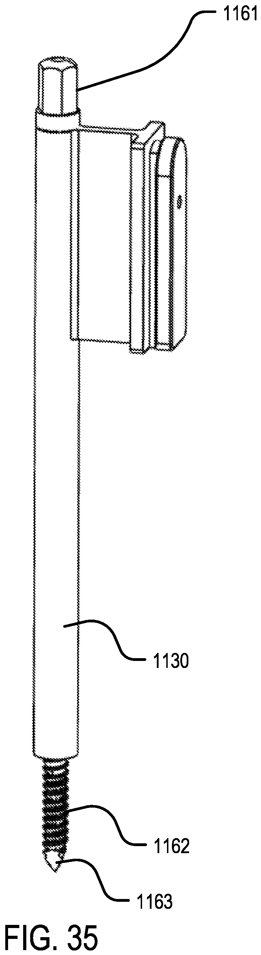

FIG. 33 is a perspective view of a pin delivery cannula 1130, FIG. 34 is a perspective view of a pin 1160 for use with the pin delivery cannula 1130 and FIG. 35 is a perspective view of the pin delivery cannula 1130 and pin 1160 in an example operative configuration. In various embodiments, the pin 1160 may be referred to as a screw, bone screw, or bone anchor, for example. In the example embodiment, pin delivery cannula 1130 may include an engagement feature 1136 having the same, similar, or substantially the same features as engagement feature 1146 explained above. Pin delivery cannula 1130 may include a cylindrical body portion 1131 extending in a proximal to distal direction that defines an internal shaft or aperture 1132 for receiving a pin 1160 therein. Pin 1160 may extend in a proximal to distal direction from a drive feature 1161 to a tip portion 1163, for example. A smooth portion 1164 of various lengths and a threaded portion 1162 may be included between drive feature 1161 and tip portion 1163. Threaded portion 1162 may comprise any type of suitable thread pattern or patterns. In other embodiments a threaded portion 1162 may not be included. As seen best in FIG. 140, pin 1160 may be inserted within the aperture 1132 of pin receiving cannula 1130. In operation, a surgeon may rotate pin 1160 within aperture 1132 thereby driving pin 1160 forward into a bone structure, for example vertebrae 1 of FIG. 30, such that the threaded portion 1162 and tip 1163 extend beyond a distal end of the pin receiving cannula and are socketed into the boney structure.

FIG. 36 is a perspective view of a support module 1150 for attaching to arm portions of various modular retractor systems disclosed herein. In the example embodiment, support module includes a ball and socket mechanism 1151 supporting a light source 1152, for example a light emitting diode (LED). In various embodiments, ball and socket mechanism may allow for the free range of movement of light source 1152 to pivot left, right, up, and down by about 5 degrees to about 15 degrees. In one embodiment, ball and socket mechanism allows for the free range of movement of light source 1152 by about six degrees. Support module 1150 may include a body portion 1159 having a slot 1157 which a coupling portion 1158 may slide forward and backward in, for example. Coupling portion 1158 may include a U-shaped clamp defining a cavity 1156, for example. A lower portion 1154 of the U-shaped clamp may include a spring tab 1155 so that a corresponding arm may be quick snapped to support module 1150 by positioning the arm within cavity 1156, for example. The support module 1150 may be secured to a corresponding arm by tightening knob 1153 which may pull the U-shaped clamp upward such that lower portion 1154 moves upward and applies a compressive force to the underside of a corresponding arm. Additionally, in various embodiments, when knob 1153 is not fully tightened the body portion 1159 may freely rotate with respect to the coupling portion and vice versa.

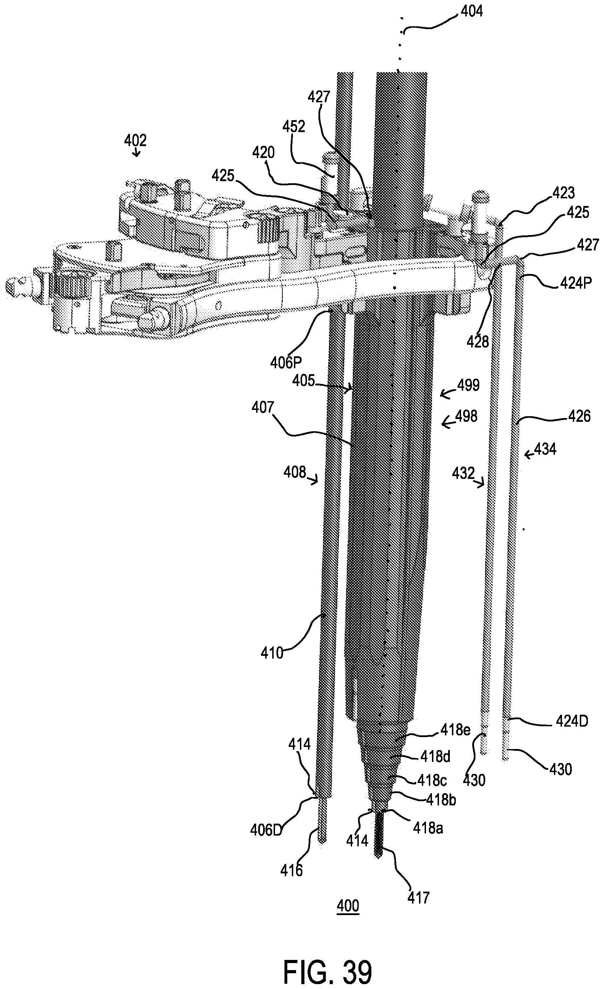

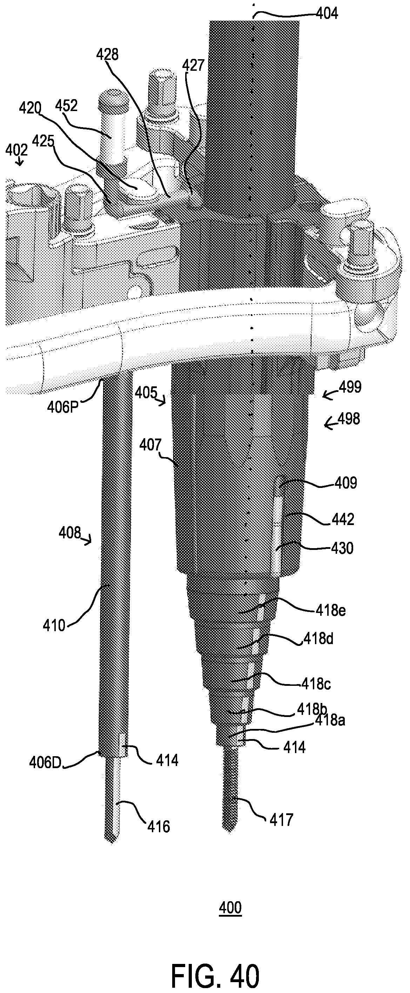

Referring to FIG. 39 and 40, an embodiment of a neuromonitoring tissue retraction system 400 is shown. The tissue retraction system 400 consists of any number of blades consistent with this disclosure. In the illustrated embodiment, a plurality of blades surround a plurality of nested dilators. As illustrated, a posterior blade 405 may be coupled to a tissue retractors 402 to facilitate creating an operative corridor in patient anatomy, *i.e.* in disc space. One posterior blade 405, a caudal blade, cranial blade, and tissue retractor 402 are shown, however, it is appreciated that the tissue retraction system 400 may include a plurality of blades and modular tissue retractor components. For example, in the illustrated embodiment three blades form the operative corridor and in other embodiments four blades may form the operative corridor. The posterior blade 405 includes a blade shaft 407 that surrounds a set of nested dilators 422. The set of nested dilators 422 includes a threaded tip 417 extending longitudinally from a first dilator 418a. The threaded tip 417 may facilitate anchoring in a disc space. The tissue retraction system 400 may also consist of an initial dilator 408. The initial dilator 408 includes a distal end 406D having a sharp trocar tip 416 extending longitudinally from the distal end 406D. Each dilator 418a-418e in the set of nested dilators 422 as well as the initial dilator include at least one exposed conductive element 414. Also shown is a free probe 432 and a locked probe 434 each oriented relative to the tissue retractor 402. The free probe 432 and/or the locked probe 434 can be fastened to the tissue retractor 402 in various ways such as via a clip or other mechanical fastener or be received in an aperture of the tissue retractor 402. In at least one embodiment, either of the probes 432, 434 extend through a cannulated passageway of the posterior blade and protrude through a cutout on the distal end of the blade where the electrode is exposed as will be further explained below. Each of the free probe 432 and locked probe 434 have a conductive tip 430 which may be electrically stimulated when in contact with muscle tissue to provide neuro monitoring capability to the tissue retraction system 400. For example, the sensing of a proximity and a relative direction to a patient nerve of interest.

As can be seen in FIG. 40, an insulated probe 409 is received in the interior of the blade shaft 407. The blade shaft 407 of posterior blade 405 includes a slot 442 configured to expose a conductive tip 430 of the insulated probe when the insulated probe 409 is received in the blade shaft 407. The conductive tip 430 may be capable of sending a signal to circuitry or other electrical device, e.g., a neuromonitoring system, upon contact with muscle tissue so as to aid the tissue retraction system 400 in neuro monitoring. The probe 409 may provide directional proximity of the entire tissue retraction system 400 during insertion as position is known in relation to other blades and dilators. In some embodiments, the insulated probe 409 may be disposable. Additionally or alternatively, the insulated probe 409 can stabilize the posterior blade 405 and/or a spinal disc during a surgical procedure. In still other embodiments, the insulated probe 409 may be replaced with a stabilization pin or similar device.

As can also be seen in FIG. 40 each of the dilators 418a-e in the set of nested dilators 422 and the initial dilator 408 include a conductive element 414. The conductive element 414 is located on the body 410 of each of the dilators (the body 410 being the circumferential curved surface forming a cylinder in the case where the dilators have a circular cross section as in FIG. 40). Conductive elements 414 are capable of active neuro monitoring and may provide an indication of nerve position e.g. distance and direction of the nerve based on variation of electrical intensity. In some embodiments, posterior blade 405 may include partially conductive features, e.g., a semiconductor and/or other passive electrical devices such as resisters, diodes, and etc. In other embodiments, posterior blade 405 may be an insulator such that it does not interfere with electrical signal processing of the electrical devices disclosed herein.

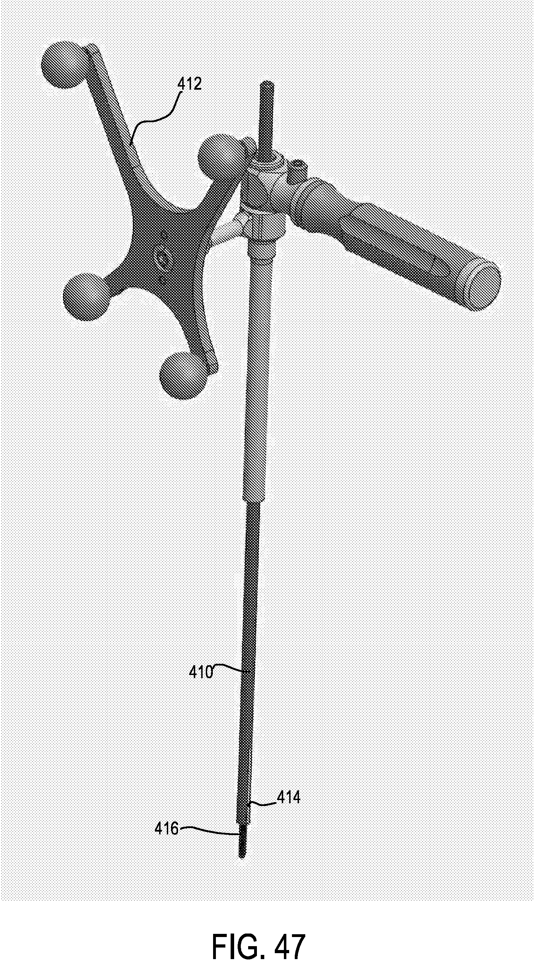

The initial dilator 408 includes a shaft extending longitudinally between a proximal end 406P and a distal end 406D. The initial dilator is received in the tissue retractor 402 at the proximal end 406P. A sharp trocar tip 416 extends longitudinally from the distal end 406D of the initial dilator 408. The trocar tip 416 is sufficiently sharp to at least partially penetrate the disc. In addition, the trocar tip 416 can stabilize a spinal disc during a surgical procedure. In some embodiments, the initial dilator 408 may include a threaded tip, such as threaded tip 417. The initial dilator 408 may be capable of neuromonitoring and/or directional monitoring. Directional monitoring may be accomplished via communication e.g. wireless, wired, Bluetooth, ethernet, near field communication, RFID, Infrared, cellular or any other communication method known to one of ordinary skill in the art between a navigation guide 412 mounted on the initial dilator 408 (shown in FIG. 47) a navigation system (not shown) or other circuitry or electrical components. The navigation guide 412 can provide the direction of the initial dilator 408 allowing targeting center of the vertebral disc regardless surrounding soft tissue structure. In addition, during insertion of the set of nested dilators 422, the dilators 418a-e can be rotated around the direction provided by the navigation guide 412. During rotation and penetration of the muscle by the initial dilator 408 and/or the set of nested dilators 422 neurologic response is monitored both in penetration (depth), direction (proximity to the nerve) related to muscle position. This allows the user to penetrate the muscle as far as possible from nerves while targeting patient anatomy e.g. the center of the disc.

In addition, the initial dilator 408 may be capable of neuro monitoring via the conductive element 414. The conductive element 414 may help guide the insertion of the dilators through muscle fiber. For example, each subsequently or iteratively larger dilator 418a-418e may be slipped over the immediately prior dilator and each dilator can transmit neuromonitoring signals as the operative corridor is sequentially enlarged. In some embodiments, conductive element 414 may communicate with an external diagnostic tool (not illustrated). The conductive element 414 may include a conductive material such as a metal like copper and be conductive and/or have terminals for electrical conduction between conductive pads placed external to tissue retraction system 100.

The dilators 418a-e in the set of nested dilators 422 are shown to have substantially circular cross sections, however, different shaped cross sections are contemplated, for example, bi-convex or elongated and substantially flat sides with convex ends, and may have a curvature but without being precisely circular or elliptical, some such embodiments having a minor axis roughly corresponding to the radius of the circular cross section. For example, the size and shape of the circular cross section of first dilator 418a may be defined by a radius extending from a center point thereof. In other embodiments, the first dilator 418a may have an ellipsis or elliptical cross section. The shape of the ellipsis or elliptical cross section may be defined by a major axis and a minor axis extending perpendicularly with respect to one another from a center point thereof. In the illustrated embodiment, the set of nested dilators 422 includes five dilators 418a-418e but other multiplicities are contemplated, *i.e.*, the set of nested dilators can include more or less dilators than five. In some embodiments, the individual dilators 418a-418e in the set of nested dilators 422 have varying cross sections which may take the form of various arrangements. In the illustrated embodiment, the dilators 418a-418e are arranged parallel with the longitudinal axis 404 of the plurality of blades defining the operative corridor, including the posterior blade 405, caudal blade 499, and cranial blade 498, in order of descending cross section with the dilator with the largest cross section (fifth dilator 418e) nearest the tissue retractor 402 and with the smallest cross section (first dilator 418a) furthest from the tissue retractor 402. For example, the dilator with the narrowest cross section may extend the farthest away from the distal end of the posterior blade 405 and each successively larger cross section dilator may extend less than the adjacent dilator exterior to it.

In some embodiments, the set of nested dilators 422 may include, for example, a second dilator 418b in addition to the first dilator 418a. The first dilator 418a and second dilator 418b may each have a major and minor axis. The second dilator 418b may be inserted around the first dilator 418a between fibers of a muscle, e.g., the paraspinous muscle, psoas muscle, such that a major axis of the second dilator 418b is initially arranged parallel with the fibers of the paraspinous muscle and can therefore be inserted around the first dilator 418a. Once inserted around the first dilator 418a, second dilator 418b can be rotated such that the major axis of second dilator 418b is perpendicular to the orientation of the fibers of the paraspinous muscle thereby gently separating the fibers by orienting the second dilator 418b such that the major axis area of the second dilator 418b gently and controllably applies pressure to separate the fibers. In other embodiments the cross sections of the first dilator 418a and second dilator 418b may form concentric circles. It is appreciated that there may be additional dilators 418c-e in the set of nested dilators 422 with similar relative arrangements to those discussed above.

Each of the dilators 418a-e in the set of nested dilators 422 may also be capable of neuro monitoring in a similar manner as the above discussed initial dilator 418a and may include the same, substantially the same, or similar conductive elements. As such, duplicative disclosure will be omitted. The set of nested dilators 422 may sequentially gently separate fibers of a muscle. The set of nested dilators 422 may be inserted sequentially and rotated on an as needed basis to gently dilate an anatomical feature.

Additionally, the initial dilator 408 and/or set of nested dilators 422 may include a positive stop feature. The positive stop feature may be formed by the relative diameters of the trocar tip 416 (or threaded tip 417 in the case of the set of nested dilators 422) and a lower surface 440. The relative diameters of the trocar tip 416/ threaded tip 417 and the lower surface 440 may be about 1.5 mm and about 3 mm respectively. In addition, the surface contact angle of the lower surface 440 of each of the initial dilator 408 and the set of nested dilators 422 with the muscle fiber may range from 0 degrees to about 45 degrees in order to allow the penetration of the dilators through muscle fibers while preventing penetration of the dilators through a spinal disk. It should also be appreciated that the threaded tip 417 can stabilize a spinal disc during a surgical procedure.

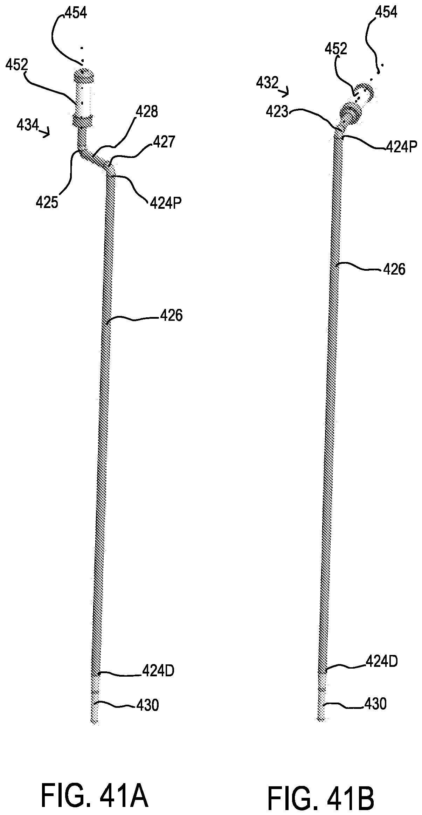

FIG. 41B shows a free probe 432. The free probe includes a cannula 426 extending longitudinally between a proximal end 424P and a distal end 424D. The free probe 432 also includes a conductive tip 430 on the distal end 424D capable of sending a signal to circuitry or other electrical component upon contact with muscle tissue so as to aid the tissue retraction system 400 (see FIG. 39 and 40) in neuro monitoring. The free probe 432 may be received in an aperture (not shown) of the tissue retractor 402 (see FIG. 39 and 40). The free probe 432 includes a first elbow 423 that forms an angle of about 90 degrees with a datum 454 of a cylindrical top portion 452. The first elbow 423 creates friction between the cannula 426 of the free probe 432 and the aperture (cannulated passageway) of the tissue retractor 402 (see FIG. 39 and 40) sufficient to limit motion of the free probe 432 within the tissue retractor 402.

FIG. 41A shows a locked probe 434. The locked probe 434 also includes a cannula 426, distal end 424D, proximal end 424P and conductive tip 430 that is the same, substantially the same, or similar to that of free probe 432 and also capable of neuro monitoring. Accordingly, duplicate disclosure will be omitted. The locked probe 434 includes a second elbow 425 forming an angle of about 90 degrees with the datum 454 of the cylindrical top portion 452. The locked probe 434 also includes a third elbow 427 forming an angle of about 90 degrees with second elbow 425. It should be noted that these angles may range between about 80 degrees to about 100 degrees. The second elbow 425 and third elbow 427 form a branch 428 that can be secured to the tissue retractor 402 (FIG. 39 and 40) with a clip 420. In some embodiments, clip 420 may be formed as a part of the retractor arm that supports the posterior blade and/or is used to lock the posterior blade to the associated posterior retractor arm. Securing of the branch 428 with the clip 420 secures the locked probe 434 in place during a surgical procedure. It should be appreciated that the free probe 432 and locked probe 434 may be used with various types of tissue retractors other than those described herein and may be received in and/or attached to such retractor systems in a similar manner (e.g. via clip 420).

It should be noted that the insulated probe 409 of FIG. 39 and 40 may include a second elbow 425, a third elbow 427 and a branch 428 similar to that of locked probe 434. Furthermore, insulated probe 409 may be secured to tissue retractor 402 by a clip 420.

The free probe 432 and/or locked probe 434 may provide directional proximity of the entire tissue retraction system 400 during insertion as position is known in relation to other blades and dilators.

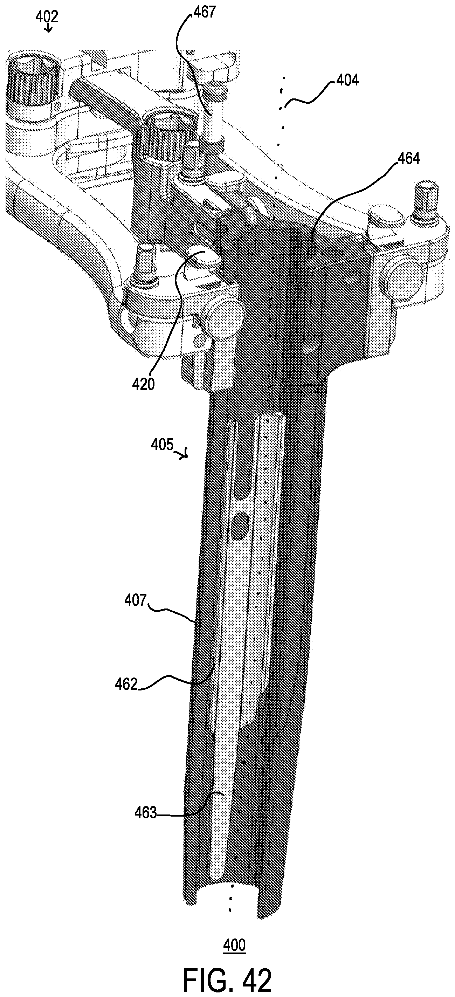

Referring now to FIG. 42, a tissue retraction system 400 with a cross sectional view of posterior blade 405 is shown. The blade shaft 407 forms a substantially cylindrical can 464 in which various components of the tissue retraction system 400 may be received. A discal shim 462 is installed on an interior surface 460 of the can 464. The shim 462 extends longitudinally along the longitudinal axis 404 of the blade shaft and laterally transversely (i.e. substantially perpendicular to the longitudinal axis 404) across the blade shaft. In the illustrated embodiment, the shim extends longer in the longitudinal direction than in the transverse direction.

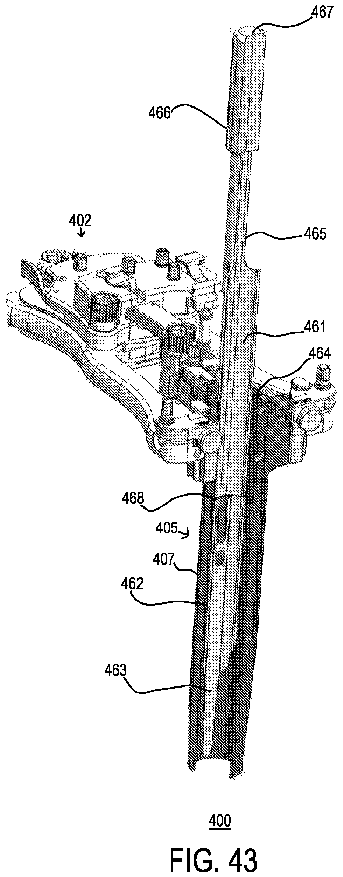

Referring now to FIG. 43, a discal shim pusher 466 is shown loaded in the can 464 of blade shaft 407. The discal shim pusher 466 has a bottom surface that can be aligned contingently with a top surface of discal shim 462 to form an interface 468. Discal shim pusher 466 also includes a stem 465 that may extend longitudinally from the can 464 so that stem 465 protrudes beyond can 464. The stem 465 further includes a top portion 467. A force may be exerted on top portion, e.g., with an impaction tool or manually so that the discal shim pusher 466 exerts a force on the discal shim 462 at the interface 468. The force exerted by the discal pusher 466 on the discal shim 462 may cause the discal shim pusher 466 to move down the longitudinal axis 404 of the posterior blade 405 away from the tissue retractor 402. This chain of force may cause discal shim tip portion 463 to extend beyond the can 464.

Discal shim 462 also includes a discal shim tip portion 463. In the illustrated embodiment, discal shim tip portion 463 includes a surface extending longitudinally from discal shim 462. The discal shim tip portion 463 can extend sufficiently far past the posterior blade so as to be inserted in patient anatomy, e.g., disc upon application of force to discal shim pusher 466.

In at least one embodiment, the discal shim 462 can be preloaded on posterior blade 405. In this embodiment, the posterior blade, cranial blade, caudal blade, etc. form the operative corridor and surround the nested dilators. Additionally, sufficient gap space exists in the posterior blade such the discal shim pusher 466 may be insert in the gap space at the proximal end of the blade to push the discal shim 462 longitudinally forward in the distal direction of the posterior blade. Likewise, the discal shim 462 is accessible when the surgeon loads the tissue retractor 402 onto the nested set of dilators 422 and/or the initial dilator 408. In addition, the disc may be stabilized with the trocar tip 422 of the initial dilator 408, the threaded tip 417 of the set of nested dilators 422 and/or the insulated probe 409. These points of contact stabilize the disc as the surgeon impacts the discal shim extendable blade 463 into the disc. This allows the surgeon to safely impact the discal shim 462 into the patient anatomy.

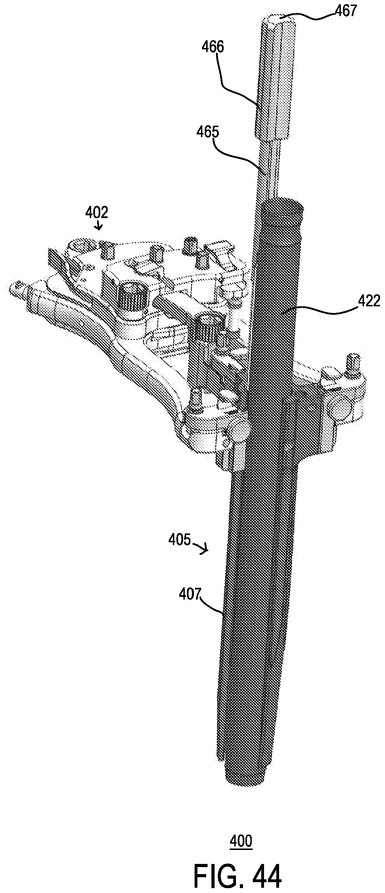

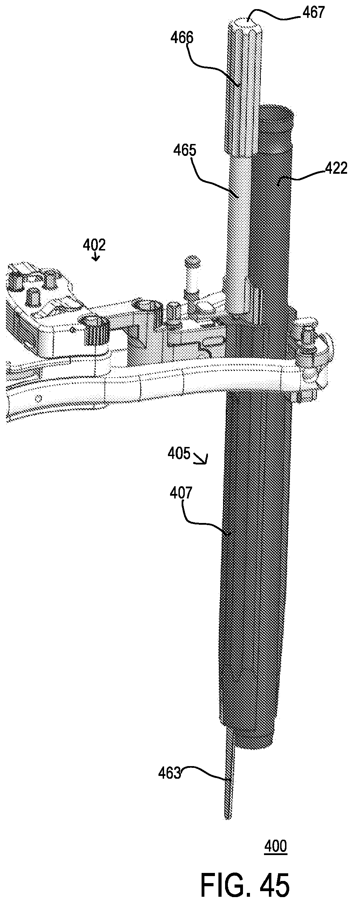

As can be best seen in FIGS. 44 and 45, the set of nested dilators 422 can be received in the can 464 of the posterior blade 405 and/or in a hollow 461 of the discal shim pusher 466 and extends along the longitudinal axis 404 of the posterior blade 405. In the illustrated embodiment, the set of nested dilators 422 extends beyond the extended length of the can.

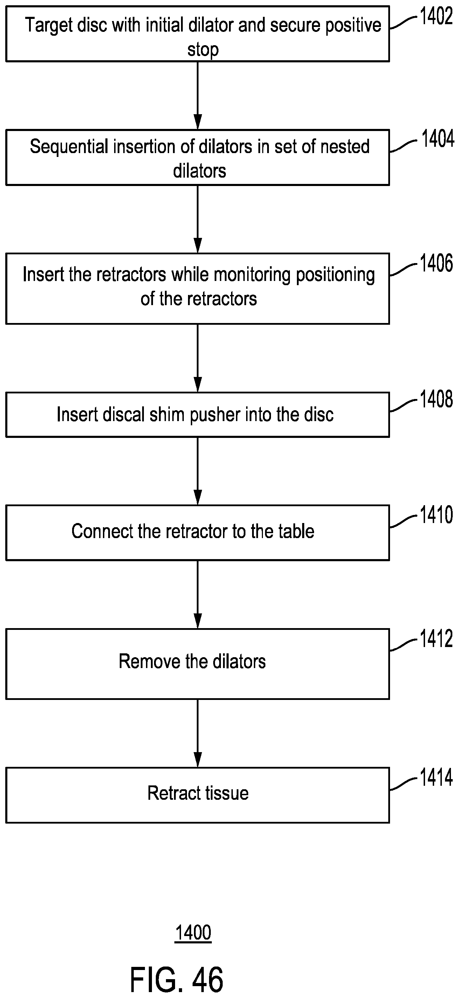

An exemplary method of use 1400 shown in FIG. 46 will now be explained. At step 1402 the user targets patient anatomy e.g. a spinal disc with the initial dilator 408 and secures the initial dilator 408 with its positive stop feature. At this point the initial dilator 408 is in contact with the patient anatomy via the trocar tip 416 (or the threaded tip 417 depending on the preference of the user) and secures the patient anatomy. The sharpness of the trocar tip 416 also allows for smooth disc penetration. In addition, directional and/or neural monitoring is in effect through the conductive element 414 and the navigation guide 412 on the initial dilator 408. At step 1404 the user sequentially inserts each of the dilators 418a-e in the set of nested dilators 422. The dilators 418 a-e can be inserted over the initial dilator 408 and be used to dilate the patient anatomy in which the initial dilator 408 is situated. The set of nested dilators 422 provides additional neuro monitoring and/or directional monitoring to the user via conductive elements 414. In addition, the threaded tip 417 on the set of nested dilators 422 provides stability to the blade and to the patient anatomy (disc). At step 1406 the user inserts one or more tissue retractors such as tissue retractor 402. Because the initial dilator 408 and set of nested dilators 422 are in place the user is able to conduct neuro monitoring and/or directional monitoring at this step and for the remainder of this method 1400 without any interruption in monitoring. This is particularly advantageous because it promotes safe tissue retraction and targeting of patient anatomy with the surgical tools. At step 1408 the user inserts discal shim extendable blade 463 into the patient anatomy using the discal shim pusher 466 as described earlier herein. At step 1410 the user may secure the one or more tissue retractors 402 of step 1406 to the surgical table thereby stabilizing the tissue retractors 402 through one or more points of contact with the surgical table. At step 1412 the user removes the initial dilator 408 and the set of nested dilators 422. The user may remove each of the dilators 418a-e in the set of nested dilators 422 all at once or sequentially e.g. one by one in the reverse order from which they were inserted. Even after the dilators has been removed the retraction system may continue to be capable of neuro monitoring via the insulated probe 409, the free probe 432 and locked probe 434 (FIG. 39). At step 1414 the user may safely retract patient tissue with the one or more tissue retractors 402 of step 1406.

Clause 1: A tissue retraction and neuro monitoring system comprising:
one or more dilators each having a conductive element, wherein the conductive element is capable of neuro monitoring, wherein at least one of the dilators includes a tip configured to penetrate patient anatomy and, wherein a distal end of each of the one or more dilators includes a surface configured to promote penetration of the one or more dilators into muscle tissue while preventing penetration of the one or more dilators into spinal disc tissue; and a first blade and a second blade, each having a respective blade shaft; a third blade having a posterior blade shaft and a slot on a lateral side thereof, the posterior blade shaft being configured to house a discal shim in a cavity thereof, wherein the discal shim is pre-loaded within the cavity of the third blade and configured to allow a user to impact the discal shim into patient disc space while the one or more dilators are positioned in nearby patient anatomy.

Clause 2: The tissue retraction and neuromonitoring system of clause 1, further comprising an insulated probe having a conductive tip, wherein the conductive tip is received in the slot of the third blade so that the conductive tip is exposed to patient anatomy and is configured to send information to an external device during a surgical procedure.

Clause 3: The tissue retraction and neuromonitoring system of any one of clauses 1-2, further comprising at least one auxiliary probe, wherein the auxiliary probe includes a conductive tip configured to send information to an external device during a surgical procedure.

Clause 4: The tissue retraction and neuromonitoring system of clause 3, further comprising a first auxiliary probe having a diameter and a bend configured to form a friction fit within an aperture of the third blade.

Clause 5: The tissue retraction and neuromonitoring system of any one of clauses 1-4, further comprising a second auxiliary probe for insertion within an aperture of the third blade and configured to be secured to the tissue retractor with a clip adjacent to an arm of the tissue retractor that supports the third blade.

Clause 6: The tissue retraction and neuromonitoring system of any one of clauses 1-5, wherein a surface of an initial dilator of the one or more dilators comprises a changing diameter and an inclined surface that is inclined between 0 degrees and about 45 degrees relative to a cross section of the one or more dilators for creating a positive stop feature to suppress the initial dilator from penetrating too far into patient anatomy.

Clause 7: The tissue retraction and neuromonitoring system of any one of clauses 1-6, further comprising a first dilator and a second dilator of the one or more dilators, wherein the first dilator includes a sharp trocar tip and the second dilator includes a threaded tip, wherein either one of the first dilator or second dilator is an initial dilator.

Clause 8: The tissue retraction and neuromonitoring system of any one of clauses 1-7, further comprising at least two dilators having a first cross section and a second cross section, respectively, wherein the first and second cross sections are arranged concentrically.

Clause 9: The tissue retraction and neuromonitoring system of clause 8, wherein the first cross section is larger than the second cross section.

Clause 10: The tissue retraction and neuromonitoring system of any one of clauses 1-9, wherein at least a portion of a shaft of each dilator of the one or more dilators is insulated,
and wherein each dilator of the one or more dilators is configured for directional monitoring of adjacent nerves including the capability of providing an indication of nerve position in distance and direction by variation of electrical intensity.

Clause 11: A tissue retraction and neuro monitoring system comprising:
one or more dilators each having a conductive element, wherein the conductive element is capable of neuro monitoring, wherein at least one of the dilators includes a tip configured to penetrate patient anatomy and, wherein a distal end of each of the one or more dilators includes a surface configured to promote penetration of the one or more dilators into muscle tissue while preventing penetration of the one or more dilators into spinal disc tissue; a first blade and a second blade, each having a respective blade shaft; a third blade having a posterior blade shaft with a slot on a lateral side thereof, the posterior blade shaft being configured to house a discal shim in a cavity thereof, wherein the discal shim is pre-loaded to allow a user to impact the discal shim into patient disc space while the one or more dilators are positioned in nearby patient anatomy, wherein the slot of the third blade is configured to receive an insulated probe having a conductive tip, so that the conductive tip is exposed to patient anatomy and is configured to send information to an external device during a surgical procedure.

Clause 12: The tissue retraction and neuromonitoring system of clause 11, further comprising a first auxiliary probe having a diameter and a bend configured to form a friction fit with an aperture of the third blade.

Clause 13: The tissue retraction and neuromonitoring system of clause 12, further comprising a second auxiliary probe configured to be secured to the tissue retractor with a clip.

Clause 14: The tissue retraction and neuromonitoring system of any one of clauses 11-13, wherein an inclined surface of each of the one or more dilators has an angle ranging between 0 degrees and 45 degrees relative to a cross section of the one or more dilators.

Clause 15: The tissue retraction and neuromonitoring system of any one of clauses 11-14, further comprising a first dilator and a second dilator, wherein the first dilator includes a sharp trocar tip and the second dilator includes a threaded tip, and wherein either one of the first dilator or second dilator is an initial dilator.

Clause 16: The tissue retraction and neuromonitoring system of any one of clauses 11-15, further comprising at least two dilators having a first cross section and a second cross section, respectively, wherein the first and second cross sections are arranged concentrically.

Clause 17: The tissue retraction and neuromonitoring system of any one of clauses 11-16, wherein at least a portion of a shaft of each dilator of the one or more dilators is insulated, and wherein each dilator of the one or more dilators is configured for directional monitoring of adjacent nerves including the capability of providing an indication of nerve position in distance and direction by variation of electrical intensity.

Clause 18: A method of using a tissue retraction and neuro monitoring system comprising: inserting an initial dilator capable and one or more probes of neuro monitoring into patient disc space; inserting at least one additional dilator capable of neuro monitoring sequentially into patient disc space; inserting one or more tissue retractors while the initial dilator and at least one additional dilator are in place in the patient disc space; impacting an extendable blade of a discal shim with a discal shim pusher; securing the one or more tissue retractors to a surgical table; removing the initial dilator and the at least one additional dilators; retracting patient tissue with the one or more tissue retractors while the one or more probes perform neuro monitoring.

Clause 19: The method of clause 18, further comprising a blade shaft housing the discal shim and an insulated probe, wherein the blade shaft includes a slot configured to receive a conductive tip of the insulated probe so that the conductive tip is exposed to patient anatomy during a surgical procedure, and the initial dilator, at least one additional dilator and the insulated probe stabilize the patient disc space as the extendable blade is impacted.

Clause 20: A tissue retraction and neuro monitoring system comprising:
one or more dilators each having a conductive element, wherein the conductive element is capable of neuro monitoring, wherein a distal end of each of the one or more dilators includes a surface configured to promote penetration of the one or more dilators into muscle tissue while preventing penetration of the one or more dilators into spinal disc tissue; and a first blade and a second blade, each having a respective blade shaft; a third blade having a posterior blade shaft and a slot on a lateral side thereof, the posterior blade shaft being configured to house a discal shim in a cavity thereof, wherein the discal shim is pre-loaded within the cavity of the third blade and configured to allow a user to impact the discal shim into patient disc space while the one or more dilators are positioned in nearby patient anatomy

Clause 21: A tissue retraction and neuro monitoring system comprising: one or more dilators each having a conductive element, wherein the conductive element is capable of neuro monitoring, wherein at least one of the dilators includes a tip configured to penetrate patient anatomy and, wherein a distal end of each of the one or more dilators includes a surface configured to promote penetration of the one or more dilators into muscle tissue; and a first blade and a second blade, each having a respective blade shaft; a third blade having a posterior blade shaft and a slot on a lateral side thereof, the posterior blade shaft being configured to house a discal shim in a cavity thereof, wherein the discal shim is pre-loaded within the cavity of the third blade and configured to allow a user to impact the discal shim into patient disc space while the one or more dilators are positioned in nearby patient anatomy.

Clause 22: A tissue retraction and neuro monitoring system comprising: one or more dilators each having a conductive element, wherein the conductive element is capable of neuro monitoring, wherein at least one of the dilators includes a tip configured to penetrate patient anatomy and, wherein a distal end of each of the one or more dilators includes a surface configured to promote penetration of the one or more dilators into muscle tissue while preventing penetration of the one or more dilators into spinal disc tissue; and a first blade and a second blade, each having a respective blade shaft; a third blade having a posterior blade shaft and an attachment to a base body on a lateral side thereof, the posterior blade shaft being configured to house a discal shim in a cavity thereof, wherein the discal shim is pre-loaded within the cavity of the third blade and configured to allow a user to impact the discal shim into patient disc space while the one or more dilators are positioned in nearby patient anatomy.

Clause 23: A tissue retraction and neuro monitoring system comprising: one or more dilators each having a conductive element, wherein the conductive element is capable of neuro monitoring, wherein at least one of the dilators includes a tip configured to penetrate patient anatomy and, wherein a distal end of each of the one or more dilators includes a surface configured to promote penetration of the one or more dilators into muscle tissue while preventing penetration of the one or more dilators into spinal disc tissue; and a first blade and a second blade, each having a respective blade shaft; a third blade having a posterior blade shaft and a slot on a side thereof, the posterior blade shaft being configured to house a discal shim in a cavity thereof, wherein the discal shim is pre-loaded within the cavity of the third blade and configured to allow a user to impact the discal shim into patient disc space while the one or more dilators are positioned in nearby patient anatomy.

Clause 24: A tissue retraction and neuro monitoring system comprising: one or more dilators each having a conductive element, wherein the conductive element is capable of neuro monitoring, wherein at least one of the dilators includes a tip configured to penetrate patient anatomy and, wherein a distal end of each of the one or more dilators includes a surface configured to promote penetration of the one or more dilators into muscle tissue while preventing penetration of the one or more dilators into spinal disc tissue; and a first blade and a second blade, each having a respective blade shaft; a third blade having a posterior blade shaft and a slot on a lateral side thereof, the posterior blade shaft being configured to house a discal shim in a cavity thereof.

Clause 25: A tissue retraction and neuro monitoring system comprising: one or more dilators each having a conductive element, wherein the conductive element is capable of neuro monitoring, wherein at least one of the dilators includes a tip configured to penetrate patient anatomy and, wherein a distal end of each of the one or more dilators includes a surface configured to promote penetration of the one or more dilators into muscle tissue while preventing penetration of the one or more dilators into spinal disc tissue; and a first blade and a second blade, each having a respective blade shaft; a third blade having a posterior blade shaft and a slot on a lateral side thereof, the posterior blade shaft being configured to house a discal shim in a cavity thereof, wherein the discal shim is configured to allow a user to impact the discal shim into patient disc space while the one or more dilators are positioned in nearby patient anatomy.

Clause 26: A tissue retraction and neuro monitoring system comprising: one or more dilators each having a conductive element, wherein the conductive element is capable of neuro monitoring, wherein a distal end of each of the one or more dilators includes a surface configured to promote penetration of the one or more dilators into muscle; and a first blade and a second blade, each having a respective blade shaft; a third blade having a posterior blade shaft and a slot on a side thereof, the posterior blade shaft being configured to house a discal shim in a cavity thereof.

## Claims

1. A tissue retraction and neuro monitoring system comprising:
one or more dilators each having a conductive element,
wherein the conductive element is capable of neuro monitoring,
wherein at least one of the dilators includes a tip configured to penetrate patient anatomy and,
wherein a distal end of each of the one or more dilators includes a surface configured to promote penetration of the one or more dilators into muscle tissue while preventing penetration of the one or more dilators into spinal disc tissue; and
a first blade and a second blade, each having a respective blade shaft;
a third blade having a posterior blade shaft and a slot on a lateral side thereof, the posterior blade shaft being configured to house a discal shim in a cavity thereof,
wherein the discal shim is pre-loaded within the cavity of the third blade and configured to allow a user to impact the discal shim into patient disc space while the one or more dilators are positioned in nearby patient anatomy.

2. The tissue retraction and neuromonitoring system of claim 1, further comprising an insulated probe having a conductive tip, wherein the conductive tip is received in the slot of the third blade so that the conductive tip is exposed to patient anatomy and is configured to send information to an external device during a surgical procedure.

3. The tissue retraction and neuromonitoring system of any one of claims 1-2, further comprising at least one auxiliary probe, wherein the auxiliary probe includes a conductive tip configured to send information to an external device during a surgical procedure.

4. The tissue retraction and neuromonitoring system of claim 3, further comprising a first auxiliary probe having a diameter and a bend configured to form a friction fit within an aperture of the third blade.

5. The tissue retraction and neuromonitoring system of any one of claims 1-4, further comprising a second auxiliary probe for insertion within an aperture of the third blade and configured to be secured to the tissue retractor with a clip adjacent to an arm of the tissue retractor that supports the third blade.

6. The tissue retraction and neuromonitoring system of any one of claims 1-5, wherein a surface of an initial dilator of the one or more dilators comprises a changing diameter and an inclined surface that is inclined between 0 degrees and about 45 degrees relative to a cross section of the one or more dilators for creating a positive stop feature to suppress the initial dilator from penetrating too far into patient anatomy.

7. The tissue retraction and neuromonitoring system of any one of claims 1-6, further comprising a first dilator and a second dilator of the one or more dilators, wherein the first dilator includes a sharp trocar tip and the second dilator includes a threaded tip,
wherein either one of the first dilator or second dilator is an initial dilator.

8. The tissue retraction and neuromonitoring system of any one of claims 1-7, further comprising at least two dilators having a first cross section and a second cross section, respectively, wherein the first and second cross sections are arranged concentrically.

9. The tissue retraction and neuromonitoring system of claim 8, wherein the first cross section is larger than the second cross section.

10. The tissue retraction and neuromonitoring system of any one of claims 1-9, wherein at least a portion of a shaft of each dilator of the one or more dilators is insulated,
and wherein each dilator of the one or more dilators is configured for directional monitoring of adjacent nerves including the capability of providing an indication of nerve position in distance and direction by variation of electrical intensity.
